# EUROPEAN PATENT APPLICATION

(11) **EP 2 835 375 A1**
(43) Date of publication of application: **11.02.2015**
(21) Application number: 13382322.9
(22) Date of filing: 09.08.2013
(51) Int. Cl.: C07F 15/04, B82Y 30/00, B82Y 40/00, C07C 251/16, C07F 1/08, C07F 3/06, C01B 31/02

(54) **Bis-salphen compounds and carbonaceous material composites comprising them**

(71) Applicant: Fundació Institut Català d'Investigació Química, 43007 Tarragona (ES); Institució Catalana de Recerca i Estudis Avançats, 08010 Barcelona (ES); Polymaterials AG, 87600 Kaufbeuren (DE)
(72) Inventor: Kleij, Arjan Willem, 43007 Tarragona (ES); Escarcega Bobadilla, Martha Verónica, 43007 Tarragona (ES); Zelada Guillen, Gustavo Adolfo, D-87600 Kaufbeuren (DE); Maier, Gerhard, D-87600 Kaufbeuren (DE)
(74) Representative: Bailey, Sam Rogerson

(57) **Abstract**

The present invention relates to compounds of formula (I) or a coordination complex thereof with a metal ion selected from the group consisting of Ni(II), cu(II), sc(III), Fe(II), Fe(III), ca(II), Co(II), co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), cd(II), Ce(III), Ce(IV), Eu(III), Yb(III), Gd(III), and Zn(II) for use as a dispersing agent for improvement of dispersion of a carbonaceous material in a dispersion medium

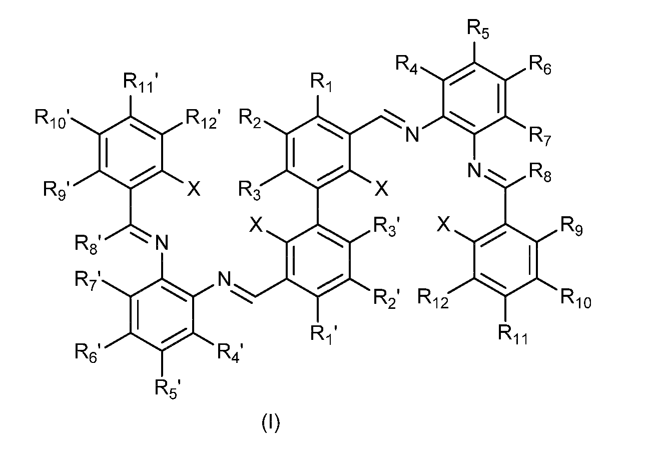

It also relates to compositions comprising a compound of formula (I), a coordination complex thereof or a mixture thereof and a carbonaceous material, and to methods of making them.

## Description

### TECHNICAL FIELD

The present proposals relate to dispersant and templating compounds for use with carbonaceous materials and composite materials incorporating these compounds with carbonaceous materials, in particular carbon nanostructures. These proposals also relate to methods of making such composite materials.

### BACKGROUND

Polymer-carbon composite materials represent an interesting and expanding family of materials with a wide range of applications due to their specific properties. Carbonaceous materials, such as carbon nanotubes, carbon black and graphite, are commonly used as additives in polymer compositions to take advantage of the beneficial properties of the carbon nanostructures and improve, mechanical, optical and/or electrical properties, among others, of the composite material. In particular, carbon nanotube-polymer composite are considered as a possible alternative to expensive and rigid indium-tin-oxide, widely used in touchscreens and various displays, among other applications. Such composites are also used in the field of performance materials since the carbon materials provide an improved mechanical resistivity to stress to the polymer matrix. Other possible applications of these materials include heat insulation, electromagnetic shielding, transparent conductive coatings, electrostatic dissipation, printed circuits and many others.

The manufacture of such composite materials however suffers from a limited processability and dispersion of the carbonaceous material in the polymeric matrix still represents one of the major challenges in the field. The carbonaceous material tends to aggregate and form clusters or bundles within the polymer matrix which reduces the homogeneity of the material and its isotropy and can impair the desirable properties of the composite. In particular, in the case of electrically conductive composite materials of carbon nanotubes, higher performance is likely obtained for well-dispersed, yet physically connected nanotubes, thus forming embedded electrically conductive networks of nanowires. Another important issue related to composites of carbonaceous materials and in particular carbon nanotubes, relates to the control of the alignment of the carbon materials at the nanoscale in a homogeneous way.

Some methods of aligning carbon nanostructures, in particular carbon nanotubes, in a polymer matrix are known. A brief overview is given in Macromolecules (2006), 39(16), 5194-5205 and includes:
- direct mixing (solution blending or melt blending) with or without a solvent. This is easy to implement but requires high loadings of carbon nanotubes (CNTs) and the technique is limited to certain polymer matrices.
- a latex procedure which involves debundling of CNTs by treatment of the CNTs with a surfactant followed by sonication, centrifugation, polymer addition and drying (Carbon 2007;45:2897-903 and US 7999028 B2).
- application of an electromagnetic field (WO 2009/035478). This method presents some difficulties for implementation and some limitations for the nanostructures used therein.
- chemically modifying the CNTs to improve dispersability in the polymer matrix (US 2012145968 and WO 2008/114910).
- Utilisation of mixed polymer matrices (WO 2008/143233)
- in-situ polymerization

All of these methods involve significant drawbacks in terms of versatility and ease of implementation. A high load of the carbonaceous material is usually required to fully incorporate and disperse the material in the polymer matrix. Also the methods are often hard to implement and are not cost-effective for use in high-volume.

There are some reports of self-assembled systems of carbonaceous nanostructures in which carbon nanotubes (CNTs) or fullerene derivatives interact with porphyrin oligomers or peptide helices to form structured assemblies at the nanoscale. However, these methods have not been demonstrated in polymer matrices or on non-metallic supports.

Some bis-salphen compounds have been developed and studied by Kleij et al. These compounds have proven useful in the molecular recognition of chiral molecules, and as a precursor of a trinuclear catalyst for the preparation of cyclic carbonates from epoxides and carbon dioxide.

There remains a need for composites in which carbonaceous materials are well dispersed and preferably aligned or interconnected in a polymer matrix, which are cost effective to produce and may offer the capability to tune the properties of the composite by varying the carbon incorporation parameters.

### Further References Cited In The Application

- Moniruzzaman et al. Macromolecules (2006), 39(16), 5194-5205
- Yu et al., Carbon 2007,45,2897-903
- US 7999028 B2
- WO 2009/035478
- US 2012145968
- WO 2008/114910
- WO 2008/143233
- Kleij et al. Chem. Eur. J. 2013, 19, 2641 - 2648
- Kleij et al. Chem. Eur. J. 2012, 18, 6805 - 6810
- Kleij et al. Dalton Trans., 2012, 41, 9766-9772
- Kämpfe et al. Eur. J. Inorg. Chem. 2009, 1027-1035
- Zhang et al. J. Org. Chem. 2001, 66, 481-487
- Görl et al. J. Organomet. Chem. 2007, 692, 5727-5753

### SUMMARY

The present proposals relate to the use of bis-salphen compounds as defined herein as dispersing agents to disperse carbonaceous materials, in particular CNTs, in a polymer, gel, solvent or onto a supporting surface.

Further proposals relate to the use of bis-salphen compounds as defined herein as templating agents to promote the self-assembly of carbonaceous materials into nanostructures in a dispersion medium.

In one aspect, the present proposals relate to a compound of formula (I) or a coordination complex thereof with a metal ion selected from the group consisting of Ni(II), Cu(II), Sc(III), Fe(II), Fe(III), Ca(II), Co(II), Co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II) for use as a dispersing agent for improvement of dispersion of a carbonaceous material in a dispersion medium 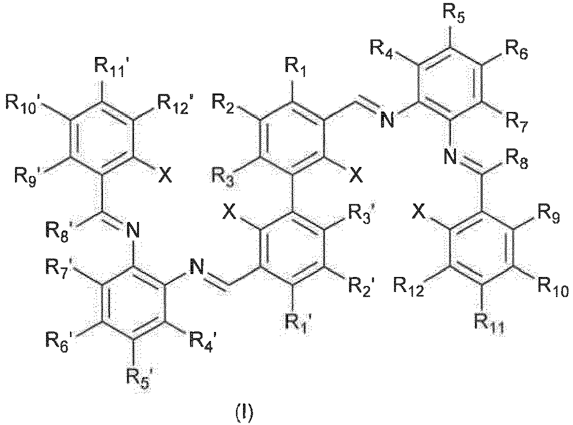 wherein:
each X is independently selected from OH and SH;
R₁, R₂, R₃, R₁', R₂' and R₃' are each independently selected from the group consisting of halogen, hydrogen, cyano and nitro; provided that one or more of the pairs of groups R₁ and R₂, R₂ and R₃, R₁' and R₂', R₂' and R₃' together with the carbon atoms to which they are attached optionally form a phenyl group or a 5 or 6 membered heteroaromatic ring having one or more heteroatoms selected from O, N and S; wherein one or more of the hydrogen atoms of the phenyl or heteroaromatic ring is optionally replaced by a radical selected from halogen, nitro and cyano;
R₄, R₅, R₆, R₇, R₉, R₁₀, R₁₁, R₁₂, R₄', R₅', R₆', R₇', R₉', R₁₀', R₁₁', and R₁₂', are each independently selected from H, halogen, nitro, cyano, C₁₋₆ alkyl, -O(C₁₋₆ alkyl), -(C=O)C₁₋₆ alkyl, -O(C=O)C₁₋₆ alkyl, -(C=O)O(C₁₋₆ alkyl), C₃₋₈ cycloalkyl, C₃₋₈ heterocycloalkyl, phenyl, and C₅₋₆ heteroaryl, wherein any of the C₃₋₈ cycloalkyl, C₃₋₈ heterocycloalkyl, phenyl, and C₅₋₆ heteroaryl groups are optionally substituted with one or more groups selected from halogen, nitro, cyano, and C₁₋₆ alkyl; provided that one or more of the pairs of groups R₄ and
R₅, R₅ and R₆, R₆ and R₇, R₉ and R₁₀, R₁₀ and R₁₁, R₁₁ and R₁₂, R₄' and R₅', R₅' and R₆', R₆' and R₇', R₉' and R₁₀', R₁₀' and R₁₁', R₁₁' and R₁₂' together with the carbon atoms to which they are attached optionally form a phenyl group or a 5 or 6 membered heteroaromatic ring having one or more heteroatoms selected from O, N and S; wherein one or more of the hydrogen atoms of the phenyl or heteroaromatic ring is optionally replaced by a radical selected from halogen, nitro, and cyano, C₁₋₆ alkyl, C₁₋₆ haloalkyl, -O(C₁₋₆ alkyl), -(C=O)C₁₋₆ alkyl, -O(C=O)C₁₋₆ alkyl, and -(C=O)O(C₁₋₆ alkyl);
R₈ and R₈' are independently selected from: a phenyl group or a 5 or 6 membered heteroaromatic ring having one or more heteroatoms selected from O, N and S, the phenyl or heteroaromatic ring optionally being bridged or fused with one or more 5 to 12 membered ring; wherein one or more of the hydrogen atoms of each ring is optionally replaced by a radical selected from halogen, nitro, and cyano.

These proposals also provide a composite material comprising: a bis-salphen compound as defined herein; and a carbonaceous material; and optionally a polymer or gel component.

These proposals also provide a method for preparing a composite material as defined herein comprising the steps of: preparing a mixture of carbonaceous material with a compound as defined herein and optionally also a polymer or gel, in a solvent; and eliminating the solvent to provide the composition.

These proposals also relate to methods of improving the dispersion of a carbonaceous material in a dispersion medium using a compound as defined herein.

These proposals further relate to the use of compounds as defined herein to improve or effect the dispersion of a carbonaceous material in a dispersion medium.

### BRIEF DESCRIPTION OF THE FIGURES

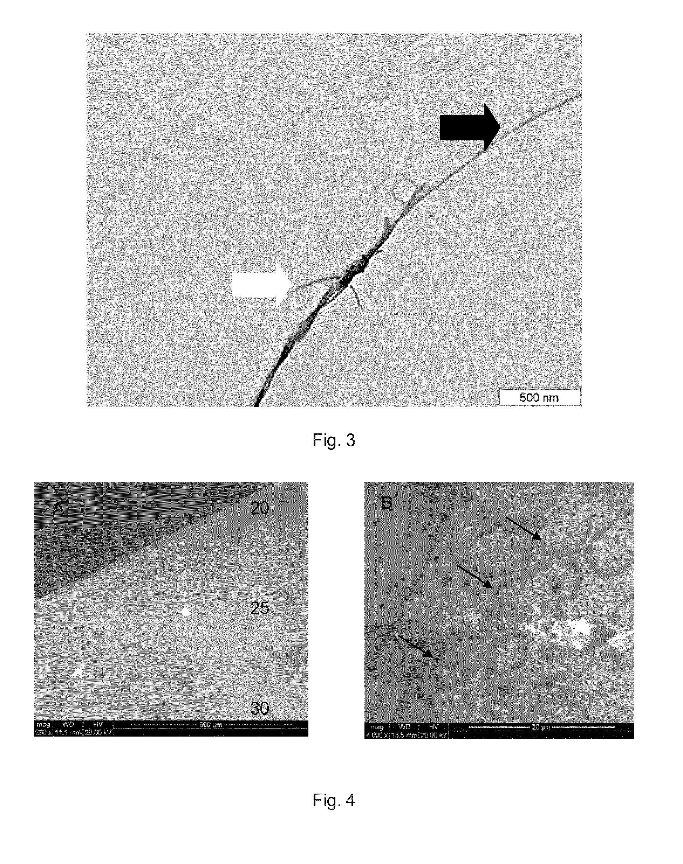

Fig. 1 shows two bis-salphen framework structures in parts A1 and A2 with the various options for the M metal centre in each being listed as a, b and c. Parts 1 a, 1 b, 1 c and 2a show TEM micrographs of solutions of compounds 1 a, 1 b, 1 c and 2a respectively in dichloromethane (DCM) after evaporation of the solvent on a flat surface consisting of a carbon-coated Formvar film.
Fig. 2 shows size distributions measured by dynamic light scattering for (x axis is diameter in nm, y axis is the peak intensity, expressed as a percentage):
   Empty black squares: solution of multi walled carbon nanotubes (MWNTs) at a 0.02 mg per mL concentration (in dichloromethane, DCM),
   Empty black circles: solution of compound 2a at 70 nM concentration in DCM Grey dashed line: solution of compound 1 a at 70 nM concentration in DCM Dashed line marked with solid black triangles: solution of compound 2a at 70 nM concentration in DCM and MWNT at 0.02 mg per mL
   Dashed line marked with crosses (X): solution of compound 1 a at 70 nM concentration in DCM and MWNT at 0.02 mg per mL
   Dashed line marked with solid black circles: solution of compound 1a at 280 nM concentration in DCM and MWNT at 0.08 mg per mL
   Dotted line marked with crosses (+); solution of compound 2a at 280 nM concentration in DCM and MWNT at 0.08 mg per mL
Fig. 3 shows a TEM micrograph (scale bar 500 nm) of a dropcast solution of complex 1a in DCM containing MWNTs. The white arrow points to nanotubes, while the black arrow shows a self-assembled rod of compound 1a.
Fig. 4 shows TEM micrographs of a blank bisphenol-A polycarbonate polymer film prepared by compression moulding (part A - scale bar 300 µm) and a similarly prepared film to that in part A but incorporating compound 2a and MWNTs (part B - scale bar 20 µm). Arrows show rings of MWNTs.

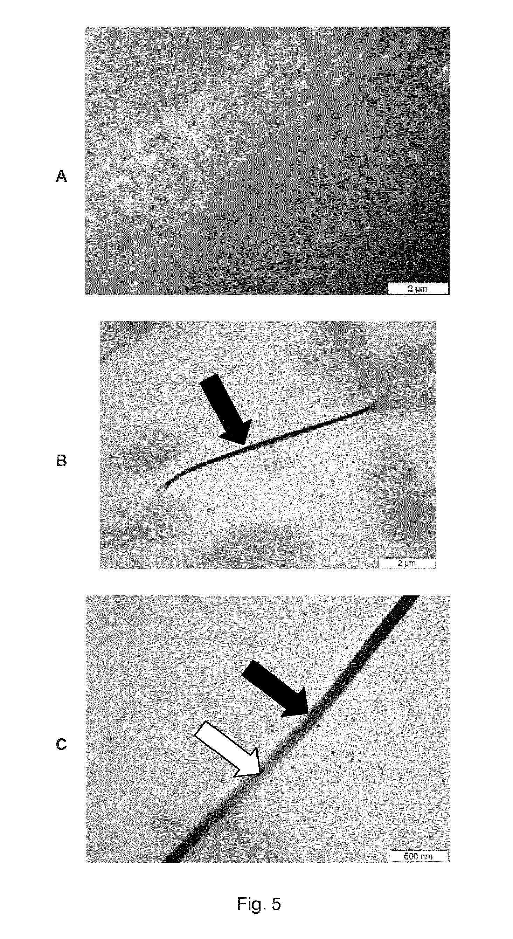

Fig. 5 shows TEM micrographs of transverse cuts of polymer films prepared by spin coating from solutions composed of bisphenol-A polycarbonate (part A - scale bar 2 µm), bisphenol-A polycarbonate with compound 2a (part B - scale bar 2 µm), and bisphenol-A polycarbonate with compound 2a and MWNTs (part C - scale bar 500 nm). In parts B and C, the black arrows points to a rod of compound 2a. In part C, the white arrow points to MWNTs embedded into a rod of compound 2a.

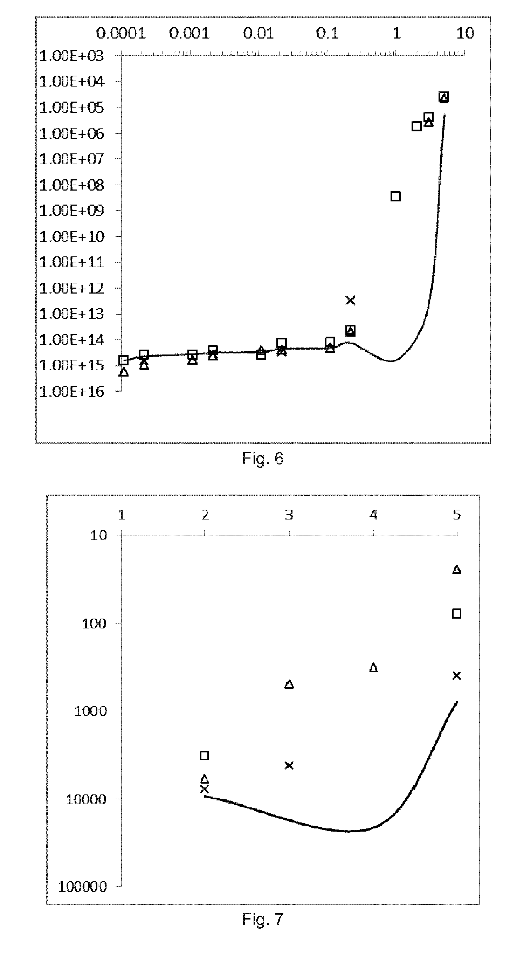

Fig. 6 shows the surface resistivity (ohm) measurements of polycarbonate films with different compositions. X axis is the load of MWNTs, expressed in weight percent and represented on a logarithmic scale. Y axis is the surface resistivity of the film.
   Solid line: (bisphenol A polycarbonate + MWNCT)
   Crosses: (bisphenol A polycarbonate + MWNCT + Compound 1 c)
   Triangles: (bisphenol A polycarbonate + MWNCT + Compound 1 a)
   Squares: (bisphenol A polycarbonate + MWNCT + Compound 2c)
Fig. 7 shows volumetric resistivity (ohm·cm) of different films of composite materials containing 0.07 wt.% bis-salphen compound as a function of the load of MWNTs (x axis), expressed in weight percent.
   Solid line: (bisphenol A polycarbonate + MWNCT)
   Crosses: (bisphenol A polycarbonate + MWNCT + Compound 2a)
   Triangles: (bisphenol A polycarbonate + MWNCT + Compound 1 a)
   Squares: (bisphenol A polycarbonate + MWNCT + Compound 1c)

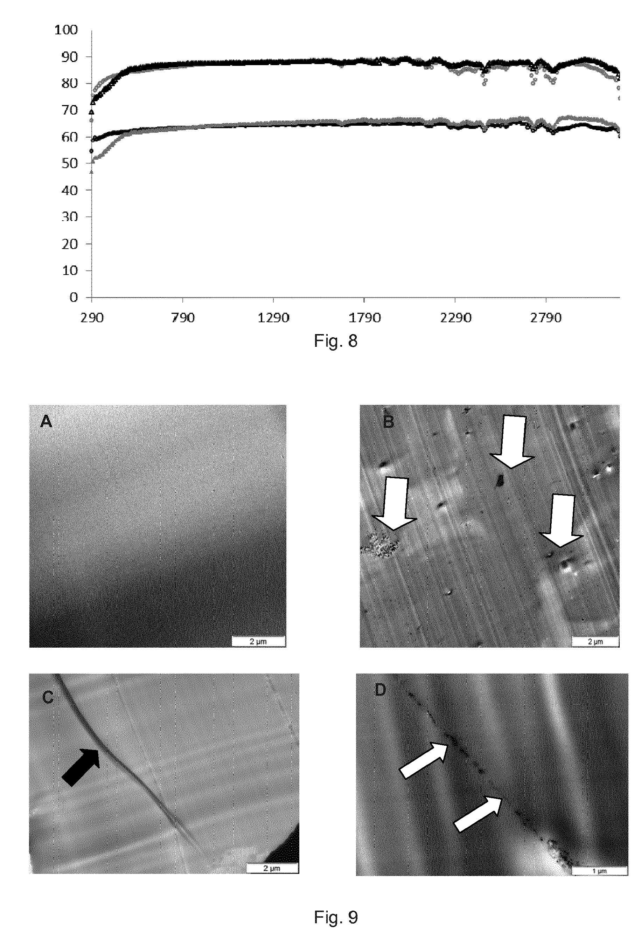

Fig. 8 shows the transmittance, expressed as a percentage (y axis), as a function of the wavelength (x axis), expressed in nm, of the following film compositions, obtained by compression moulding:
   Black triangles (top trace): bisphenol A polycarbonate
   Grey circles (top trace): bisphenol A polycarbonate+0.07 wt% of compound 2a
   Grey triangles (bottom trace): bisphenol A polycarbonate+0.5 wt%
   MWNTs+0.07 wt% compound 2a
   Black circles (bottom trace): bisphenol A polycarbonate+0.5 wt% MWNTs.
Fig. 9 shows TEM images of parts obtained by melt extrusion and injection moulding of: A) Bisphenol A polycarbonate (BPA), scale bar 2 µm; B) BPA + 0.3 weight % MWNT (scale bar 2 µm), white arrows point MWNT aggregates; C) BPA + compound 1 a (0.07 weight%), scale bar 2 µm, black arrow points to a nanorod of 1 a; D) BPA + MWNT (0.3 weight%) + compound 1 a (0.07 weight %); scale bar 1 µm; white arrows point to nanotubes aligned along a rod of compound 1 a.

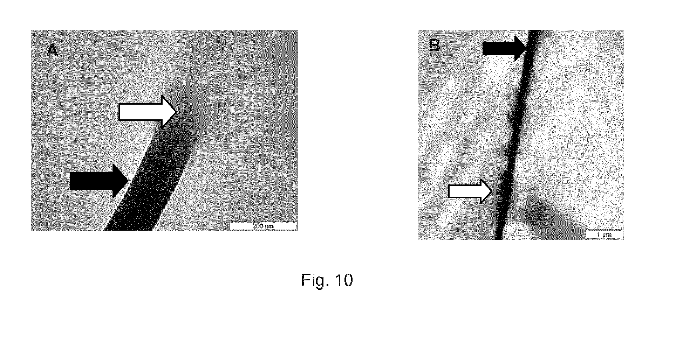

Fig. 10 shows TEM images of films prepared by compression moulding of: A) bisphenol A polycarbonate, compound 1 a (0.07 weight%) and MWNT (0.02 weight%), scale-bar 200 nm; B) bisphenol A polycarbonate, compound 1 a (0.07 weight%) and MWNT functionalized with carboxyl groups (0.02 weight%), scale-bar 1 µm. The black arrows points to a nanorod of compound 1 a. The white arrow points to MWNTs embedded into a nanorod of compound 1 a.

### FURTHER DEFINITIONS; OPTIONS AND PREFERENCES

In the context of the invention, the term C₁₋₆ alkyl refers to a saturated, linear or branched aliphatic hydrocarbon radical containing from 1 to 6 carbon atoms. Non limitative examples include methyl, ethyl, propyl, isopropyl, n-butyl, tert-butyl, sec-butyl, pentyl, neo-pentyl.

In the context of the invention, the term "dispersing agent" in relation to a carbonaceous material refers to a compound which improves the dispersion of the carbonaceous material in a dispersion medium. The dispersion medium is as defined herein and may preferably be a polymer, gel or liquid medium, such as a halogenated liquid. This improvement in dispersion may involve preventing the spontaneous aggregation of the carbonaceous material particles into packed arrangements, e.g. bundles, or promoting the dispersion of bundles of the carbonaceous material into the dispersion medium. The carbonaceous material or dispersion optionally being placed onto a surface or within a volume. Compositions incorporating such a dispersing agent may show a more uniform distribution of carbonaceous material throughout the dispersion medium than a comparable composition that does not contain the dispersing agent.

In the context of the invention, the term "dispersion medium" refers to a continuous medium, such as a gas, a liquid or a solid in which a disperse phase is distributed. Examples of dispersion media are defined herein and may include liquid dispersion media such as non-aqueous or aqueous solvents, including halogenated solvents and more particularly, chlorinated solvents such as dichloromethane, chloroform, tetrachloromethane and tetrachloroethane. Non-limiting examples of solid dispersion media include polymeric materials, ceramics, gel components, and silica. For example the dispersion medium may be selected from any of the liquid, gel, polymer or solid media described herein.

In the context of the invention, the term C₅₋₆ heteroaryl refers to an aromatic ring containing from 5 to 6 atoms selected from the group consisting of C, N, O and S.

The bis-salphen compounds defined herein are compounds of formula (I) which may be complexed with a metal ion selected from Ni(II), Cu(II), Sc(III), Fe(II), Fe(III), Ca(II), Co(II), Co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II).

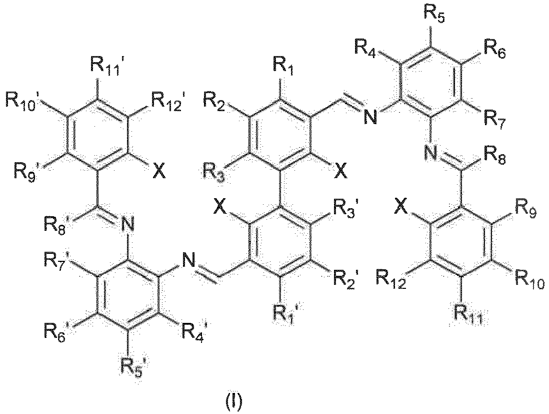

In the context of the invention, a coordination complex of a compound of formula (I) with a metal refers to a compound of formula (I) wherein one or more metal atom is bonded to two X and two N atoms of the compounds of formula (I), with the proviso that two N atoms bonded to the same metal atom are born by the same aromatic ring and that the X and N atoms bonded to the same metal atom are separated by four covalent bonds. For the metals in the oxidation state +2, the geometry of the metal may be square planar. For some metals, the coordination complex may further comprise one or two axial ligands, in which the metal atom is bonded to the axial ligand orthogonal to the plane formed by the metal and the two nitrogen atoms of formula (I) to which the metal is bonded. Non limitative examples of such axial ligands are halide, carbon monoxide, aquo, hydroxy, cyanide, phosphines, phosphites, carboxylates, aromatic heterocycles, alkoxides, and aryloxides.

The metal ions with which the bis-salphen compounds of formula (I) may be complexed are preferably independently selected from Ni(II), Cu(II), Sc(III), Fe(II), Fe(III), Ca(II), Co(II), Co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II). Preferably, the metal ions are selected from Ni(II), Cu(II), Fe(II), Co(II), Mn(II), Cr(II), Ti(II),Pd (II), Ru (II), Mn(II), In(II), Sn(II), Mg(II) and Zn(II). More preferably the metal ions are selected from Ni(II), Cu(II) and Zn(II).

In some aspects of the compounds described herein, each X is independently selected from OH and SH, preferably OH.

In some aspects of the present compounds, the R₁, R₂, R₃, R₁', R₂' and R₃' groups and the groups formed when R₁ and R₂, R₂ and R₃, R₁' and R₂', or R₂' and R₃' together with the carbon atoms to which they are attached join to form a ring group, are all independently selected from planar groups, i.e. chemical groups in which the atoms are coplanar.

In preferred aspects, one of the pairs of groups R₁ and R₂ or R₂ and R₃, and, in addition, or as an alternative thereto, one of the pairs of groups R₁' and R₂' or R₂' and R₃', together with the carbon atoms to which they are attached form a phenyl group or a 5 or 6 membered heteroaromatic ring having one or more heteroatoms selected from O, N and S, with any remaining, i.e. not joined in a ring, of the groups R₁, R₂, R₃, R₁', R₂', R₃' being as defined above.

In preferred aspects, R₁ and R₁' are both hydrogen.

In preferred aspects, R₁, R₁', R₂, R₂', R₃ and R₃' are all hydrogen.

In most preferred aspects, R₁ and R₁' are both hydrogen and the pairs of groups R₂ and R₃, R₂' and R₃' together with the carbon atoms to which they are attached each form a phenyl ring.

In the compounds described herein, R₈ and R₈' are each independently selected from: a phenyl group or a 5 or 6 membered heteroaromatic ring having one or more heteroatoms selected from O, N and S, the phenyl or heteroaromatic ring optionally being bridged or fused with one or more 5 to 12 membered ring; wherein one or more of the hydrogen atoms of each ring is optionally replaced by a radical selected from halogen, nitro, and cyano. Preferably R₈ and R₈' are selected from furyl, pyrrolydyl, thiophenyl, phenyl, naphthyl, biphenylyl, and pyridyl. More preferably, R₈ and R₈' are selected from phenyl, naphthyl, and biphenylyl.

In some preferred compounds R₈ and R₈' are preferably identical groups. In preferred compounds R₈ and R₈' are both phenyl groups.

In the compounds described herein, R₄, R₅, R₆, R₇, R₉, R₁₀, R₁₁, R₁₂, R₄', R₅', R₆', R₇', R₉', R₁₀', R₁₁', and R₁₂' are each independently selected from H, halogen, nitro, cyano, C₁₋₆ alkyl, -O(C₁₋₆ alkyl), -(C=O)C₁₋₆ alkyl, -O(C=O)C₁₋₆ alkyl, -(C=O)O(C₁₋₆ alkyl), C₃₋₈ cycloalkyl, C₃₋₈ heterocycloalkyl, phenyl, and C₅₋₆ heteroaryl, wherein any of the C₃₋₈ cycloalkyl, C₃₋₈ heterocycloalkyl, phenyl, and C₅₋₆ heteroaryl groups are optionally substituted with one or more groups selected from halogen, nitro, cyano, and C₁₋₆ alkyl; provided that one or more of the pairs of groups R₄ and R₅, R₅ and R₆, R₆ and R₇, R₉ and R₁₀, R₁₀ and R₁₁, R₁₁ and R₁₂, R₄' and R₅', R₅' and R₆', R₆' and R₇', R₉' and R₁₀', R₁₀' and R₁₁', R₁₁' and R₁₂' together with the carbon atoms to which they are attached optionally form a phenyl ring or a 5 or 6 membered heteroaromatic ring having one or more heteroatoms selected from O, N and S; wherein one or more of the hydrogen atoms of the aromatic or heteroaromatic ring is optionally replaced by a radical selected from halogen, nitro, and cyano, C₁₋₆ alkyl, C₁₋₆ haloalkyl, -O(C₁₋₆ alkyl), -(C=O)C₁₋₆ alkyl, -O(C=O)C₁₋₆ alkyl, and - (C=O)O(C₁₋₆ alkyl).

Preferably R₄, R₅, R₆, R₇, R₉, R₁₀, R₁₁, R₁₂, R₄', R₅', R₆', R₇', R₉', Rio', R₁₁', R₁₂' are each independently selected from H, halogen, nitro, cyano, phenyl, and C₅₋₆ heteroaryl, wherein any of the phenyl and C₅₋₆ heteroaryl groups are optionally substituted with one or more groups selected from halogen, nitro, cyano, and C₁₋₆ alkyl; provided that one or more of the pairs of groups R₄ and R₅, R₅ and R₆, R₆ and R₇, R₉ and R₁₀, R₁₀ and R₁₁, R₁₁ and R₁₂, R₄' and R₅', R₅' and R₆', R₆' and R₇', R₉' and R₁₀', R₁₀' and R₁₁', R₁₁' and R₁₂' together with the carbon atoms to which they are attached optionally form a phenyl ring or a 5 or 6 membered heteroaromatic ring having one or more heteroatoms selected from O, N and S; wherein one or more of the hydrogen atoms of the aromatic or heteroaromatic ring is optionally replaced by a radical selected from halogen, nitro, and cyano.

More preferably R₄, R₅, R₆, R₇, R₉, R₁₀, R₁₁, R₁₂, R₄', R₅', R₆', R₇', R₉', R₁₀', R₁₁', R₁₂' are each independently selected from H, halogen, nitro, cyano, phenyl, and C₅₋₆ heteroaryl, wherein any of the phenyl and C₅₋₆ heteroaryl groups are optionally substituted with one or more groups selected from halogen, nitro, cyano, and C₁₋₆ alkyl.

Even more preferably R₄, R₅, R₆, R₇, R₉, R₁₀, R₁₁, R₁₂, R₄', R₅', R₆', R₇', R₉', R₁₀', R₁₁', R₁₂' are each independently selected from H, halogen, nitro, cyano, phenyl, and C₅₋₆ heteroaryl.

Even more preferably R₄, R₅, R₆, R₇, R₉, R₁₀, R₁₁, R₁₂, R₄', R₅', R₆', R₇', R₉', R₁₀', R₁₁', R₁₂' are each independently selected from H, halogen, nitro, and cyano.

Most preferably R₄, R₅, R₆, R₇, R₉, R₁₀, R₁₁, R₁₂, R₄', R₅', R₆', R₇', R₉', R₁₀', R₁₁', R₁₂' are each independently H.

Preferably the compounds described herein are compounds of formula II or coordination complexes thereof with a metal ion selected from the group consisting of Ni(II), Cu(II), Sc(III), Fe(II), Fe(III), Ca(II), Co(II), Co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II).

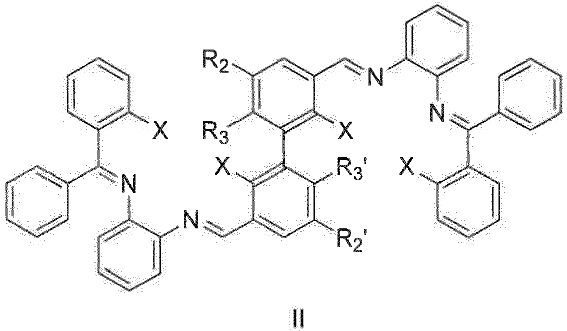

More preferably, the compounds described herein are compounds of formula II or coordination complexes thereof with a metal ion selected from the group consisting of Ni(II), Cu(II), Fe(II), Fe(III), Co(II), Co(III), Al(III), Mn(IV), Mn(II), Cr(III), Ti(II), Ti(IV), Pd(II), Ru(II), In(II), In(III), Sn(II), Sn(IV), Mg(II) and Zn(II).

In formula II, the groups X, R₂, R₃, R₂' and R₃' and preferred options for these are as defined herein. Compounds of formula (II) are compounds of formula (I) wherein R₈ and R₈' are each phenyl, and R₁, R₁', R₄, R₅, R₆, R₇, R₉, R₁₀, R₁₁, R₁₂, R₄', R₅', R₆', R₇', R₉', R₁₀', R₁₁', R₁₂' are each hydrogen.
Preferably the compounds are metal-ion complexes of formula III.

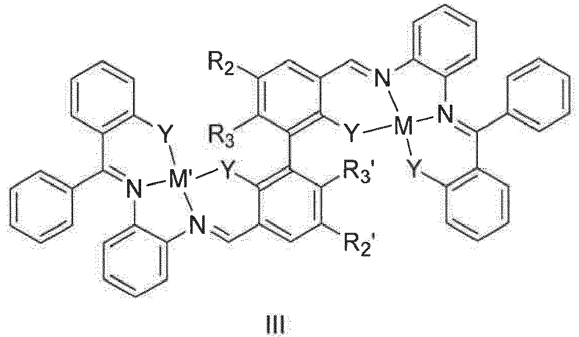

In formula III, the groups R₂, R₃, R₂', R₃' and preferred options for these are as defined herein. Each Y is independently selected from O and S, and is preferably O.
M and M' are independently selected from Ni(II), Cu(II), Sc(III), Fe(II), Fe(III), Ca(II), Co(II), Co(III), Os(III), Os(IV), Al(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II) in which ions with a coordination number greater than 4 also have one or more axial ligands selected from halide, carbon monoxide, aquo, hydroxy, cyanide, phosphines, phosphites, carboxylates, aromatic heterocycles, alkoxides, and aryloxides. Preferably M and M' are each independently selected from Ni(II), Cu(II), Fe(II), Co(II), Mn(II), Cr(II), Ti(II), Pd (II), Ru (II), Mn(II), In(II), Sn(II), Mg(II) and Zn(II), more preferably Ni(II), Cu(II) and Zn(II). Compounds of formula (III) are coordination complexes of the compounds of formula (II) with any of the metal ions M and M' mentioned above.

Preferably, the bis-salphen compounds described herein are selected from the compounds of formula 1, 1a, 1b, 1 c, 2, 2a, 2b and 2c.

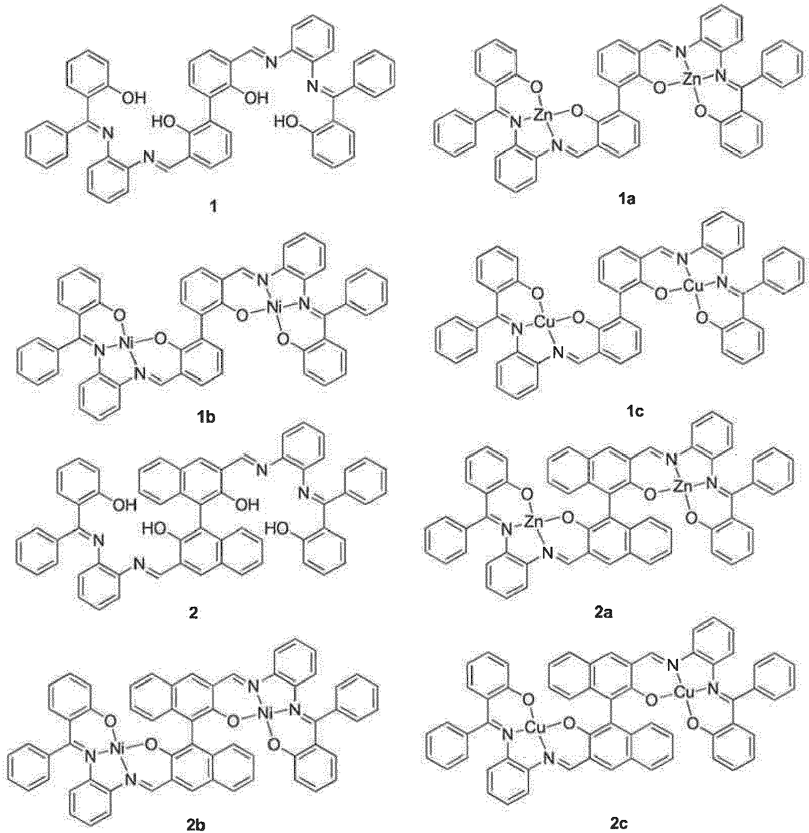

When compounds according to the present proposals are used for dispersion of a carbonaceous material, a good dispersion can be achieved, particularly dispersion of carbon nanotubes (e.g. single walled carbon nanotubes or multi walled carbon nanotubes). In particular, the carbonaceous material is dispersed evenly through the dispersion medium and, where carbon nanotubes (CNTs) are used as the carbonaceous material, the nanotubes are well separated, even to the extent of separated into individual tubes, from the bundles that are typically found in raw carbon nanotube products. In particular, the dispersion medium may be a liquid (such as halogenated solvent), a polymer, or a gel component.

Furthermore, when compounds according to the present proposals are used to disperse CNTs, the CNTs are aligned (e.g. by self-assembly) such that they are in electrically conductive contact in the polymer matrix over an extended network of CNTs.

In the context of the invention, a templating agent or templating compound for a carbonaceous material refers to a compound that promotes spontaneous organisation of the carbonaceous material within a medium into organised structures, e.g. structures that are not formed in the absence of the templating compound. For example, the compounds of the invention act as templating compounds in this way by promoting formation of interconnected rings and/or wires of the carbonaceous materials within the medium. The medium may be, in a non-limitative way, a solvent, a polymer or a gel component, for example, in some aspects the medium may be a dispersion medium as defined herein.

Dispersions of the carbonaceous material in a medium may then be deposited on a surface after which deposition, the medium, e.g. solvent, may be removed (e.g. evaporated) to leave a deposition of the carbonaceous material on the surface, preferably well dispersed and aligned in a similar manner to that in the medium.

The even, thorough dispersions of the carbonaceous material throughout a polymer, gel, or solvent or on a surface that are achievable using the present compounds means that high electrical conductivity can be achieved at relatively low loading of carbonaceous material. This low loading of the carbonaceous material can result in a high transparency of the polymer product while still maintaining a high electrical conductivity. In some cases, polymer composite products can be formed that are transparent to visible light (and may also be transparent to other wavelength radiation such as infrared radiation - although many bis-salphen compounds are typically absorbent in the ultraviolet region). Such products are particularly useful in optoelectronic devices. In some aspects, the spontaneous organisation of the carbonaceous material into organised structures (e.g. wires) as noted above allows sufficient carbonaceous material to be incorporated into the medium, e.g. polymer, gel or solvent, to increase the electrical conductivity but while still maintaining good optical transparency. Similarly, the dispersion pattern of the carbonaceous material within the medium achievable using the present compounds may allow improvement of other properties of the material, including, in a non-limitative way, magnetic shielding properties and mechanical strength.

The present proposals also relate to compositions and composite materials comprising:
a bis-salphen compound as defined herein or mixtures thereof; and
a carbonaceous material or mixtures thereof.

In some embodiments, the present proposals relate to compositions comprising:
a compound selected from the compounds of formula (I), the coordination complexes of the compounds of formula (I) and mixtures thereof; and
a carbonaceous material or mixtures thereof.

In some embodiments, the present proposals relate to compositions comprising:
a compound selected from the compounds of formula (I), the coordination complexes of the compounds of formula (I) and mixtures thereof; wherein R₁, R₂, R₃, R₄, R₅, R₆, R₇, R₈, R₉, R₁₀, R₁₁, R₁₂, R₁', R₂', R₃', R₄', R₅', R₆', R₇', R₈', R₉', R₁₀', R₁₁', R₁₂' and X are as defined in the options and preferences herein; and
a carbonaceous material or mixtures thereof.

In some embodiments, the present proposals relate to compositions comprising:
a compound selected from the compounds of formula (II), the compounds of formula (III) and mixtures thereof wherein M, M', X, Y, R₂, R₃, R₂' and R₃' are as defined in the options and preferences herein; and
a carbonaceous material or mixtures thereof.

In some embodiments, the present proposals relate to compositions comprising:
a compound selected from the compounds of formula 1, 1 a, 1 b, 1 c, 2, 2a, 2b and 2c; and
a carbonaceous material or mixtures thereof.

Such compositions may exhibit good electrical conductivity. Furthermore, these compositions may be transparent (e.g. to visible light, infrared radiation and/or ultraviolet radiation).

The combination of transparency and electrical conductivity is particularly preferred because it is these compositions that offer a possible substitute for Indium-Tin-Oxide (ITO) in optoelectronic applications.

In the context of the invention, a carbonaceous material refers to a material that is or derives from the allotropic forms of carbon: amorphous carbon, graphite, diamond, graphene, fullerenes and nanotubes. The material may further comprise a doping agent (e.g. metal atoms, nitrogen atoms, boron atoms) or may further be functionalized with various functional groups, such as carboxyl, halogen, hydroxy, esters and amine.

In preferred compositions the carbonaceous material is selected from carbon nanostructures. For example, the carbonaceous material is preferably selected from graphite, amorphous carbon (e.g. carbon black), graphene, graphene flakes, fullerenes, carbon nanotubes, carbon nanowires and carbon nanorods. In most preferred compositions, the carbonaceous material is selected from amorphous carbon, fullerenes and carbon nanotubes, and is preferably carbon nanotubes. The carbon nanotubes may be single-walled nanotubes, or multi-walled nanotubes (e.g. double-walled nanotubes or nanotubes with greater than two concentric tubes), or a mixture thereof.

Single-walled nanotubes (SWNTs) may be preferred because they offer a good balance between electrical conductivity and transparency, they are easier to solubilize compared to multi-walled nanotubes and their work function directly depends on diameter and chiral angle so the electrical properties are more predictable. However, SWNTs are typically more difficult and expensive to produce. Therefore, in some compositions, multi-walled nanotubes (MWNTs) are preferred. Furthermore, it is found that good transparency is demonstrated by the present compositions even when MWNTs are used as the carbonaceous material, possibly because the MWNTs are highly dispersed in the polymer by the bis-salphen compound.

The carbonaceous material may be surface-modified, e.g. treated to provide pendant surface functional groups. Some preferred functional groups may be carboxylic acid, halogen, hydroxy, alkoxy, siloxy, esters, amide or amine. Where the carbonaceous material is carbon nanotubes, they are preferably oxidised to provide carboxylic acid groups at defect sites in the tube structure and/or at the ends of the tubes. Preferably, CNTs (in particular MWNTs) may be treated (e.g. by reflux) in nitric acid which has the result of opening the ends of the nanotubes and functionalising at least the open ends with carboxylic acid groups. Standard methods for the functionalization of carbon nanotubes with functional groups such as hydroxy, amine, amide, alkoxy, siloxy, ester or halogen are well known in the art.

Thus, in some embodiments, the carbonaceous material is selected from amorphous carbon, graphite, graphene, fullerene, carbon nanorods and carbon nanotubes, being the carbonaceous material optionally functionalised with one or more groups selected from hydroxy, carboxy, halogen, alkoxy, siloxy, ester, amide and amine. In preferred embodiments, the carbonaceous material is selected from graphite, graphene, and carbon nanotubes, being the carbonaceous material optionally functionalised with one or more groups selected from hydroxy, carboxy, and halogen. When graphene is used as carbonaceous material, it may further be doped with metal atoms (e.g. aluminium, silver, copper, gold, platinum) or non-metal atoms (e.g. nitrogen, boron, fluorine). In more preferred aspects, the carbonaceous material is carbon nanotubes optionally functionalised with one or more carboxy groups. When carbon nanotubes are used as carbonaceous material, they may further be doped with non-metal atoms (e.g. nitrogen, boron).

In some aspects, the present proposals relate to compositions comprising:
a compound selected from the compounds of formula (I), the coordination complexes of the compounds of formula (I) and mixtures thereof; and
a carbonaceous material selected from amorphous carbon, graphite, graphene, and carbon nanotubes, being the carbonaceous material optionally functionalised with one or more groups selected from hydroxy, carboxy, and halogen or mixtures thereof.

In some aspects, the present proposals relate to compositions comprising:
a compound selected from the compounds of formula (I), the coordination complexes of the compounds of formula (I) and mixtures thereof; wherein R₁, R₂, R₃, R₄, R₅, R₆, R₇, R₈, R₉, R₁₀, R₁₁ R₁₂, R₁', R₂', R₃', R₄', R₅', R₆', R₇', R₈', R₉', R₁₀', R₁₁', R₁₂' and X are as defined in the options and preferences herein; and
a carbonaceous material selected from amorphous carbon, graphite, graphene, and carbon nanotubes, being the carbonaceous material optionally functionalised with one or more groups selected from hydroxy, carboxy, and halogen or mixtures thereof.

In some embodiments, the present proposals relate to compositions comprising:
a compound selected from the compounds of formula (II), the compounds of formula (III) and mixtures thereof wherein M, M', X, Y, R₂, R₃, R₂' and R₃' are as defined in the options and preferences herein; and
a carbonaceous material selected from amorphous carbon, graphite, graphene, and carbon nanotubes, being the carbonaceous material optionally functionalised with one or more groups selected from hydroxy, carboxy, and halogen or mixtures thereof.

In some embodiments, the present proposals relate to compositions comprising:
a compound selected from the compounds of formula 1, 1 a, 1 b, 1 c, 2, 2a, 2b and 2c; and
a carbonaceous material selected from amorphous carbon, graphite, graphene, and carbon nanotubes, being the carbonaceous material optionally functionalised with one or more groups selected from hydroxy, carboxy, and halogen or mixtures thereof.

In some embodiments, the present proposals relate to compositions comprising:
a compound selected from the compounds of formula (I), the coordination complexes of the compounds of formula (I) and mixtures thereof; and
a carbonaceous material that is carbon nanotubes, being the carbonaceous material optionally functionalised with one or more carboxy groups.

In some embodiments, the present proposals relate to compositions comprising:
a compound selected from the compounds of formula (I), the coordination complexes of the compounds of formula (I) and mixtures thereof; wherein R₁, R₂, R₃, R₄, R₅, R₆, R₇, R₈, R₉, R₁₀, R₁₁, R₁₂, R₁', R₂', R₃', R₄', R₅', R₆', R₇', R₈', R₉', R₁₀', R₁₁', R₁₂' and X are as defined in the options and preferences herein; and
a carbonaceous material that is carbon nanotubes, being the carbonaceous material optionally functionalised with one or more carboxy groups.

In some embodiments, the present proposals relate to compositions comprising:
a compound selected from the compounds of formula (II), the compounds of formula (III) and mixtures thereof wherein M, M', X, Y, R₂, R₃, R₂' and R₃' are as defined in the options and preferences herein; and
a carbonaceous material that is carbon nanotubes, being the carbonaceous material optionally functionalised with one or more carboxy groups.

In some embodiments, the present proposals relate to compositions comprising:
a compound selected from the compounds of formula 1, 1 a, 1 b, 1 c, 2, 2a, 2b and 2c; and
a carbonaceous material that is carbon nanotubes, being the carbonaceous material optionally functionalised with one or more carboxy groups.

In further embodiments, in the compositions described above, the weight ratio of the compound selected from the compounds of formula (I), the coordination complexes of the compounds of formula (I) and mixtures thereof to the carbonaceous material is from 1:100,000 to 1000:1. In some aspects, the weight ratio of the compound selected from the compounds of formula (I), the coordination complexes of the compounds of formula (I) and mixtures thereof to the carbonaceous material is from 1:10,000 to 500:1. In some aspects, the weight ratio of the compound selected from the compounds of formula (I), the coordination complexes of the compounds of formula (I) and mixtures thereof to the carbonaceous material is from 1:1000 to 150:1. Most preferably, the weight ratio of the compound selected from the compounds of formula (I), the coordination complexes of the compounds of formula (I) and mixtures thereof to the carbonaceous material is from 2:1 to 1:60.

In some aspects, the present compositions further comprise a polymer or gel component. Preferably, the carbonaceous material and the compound as described herein is dispersed in the polymer or gel component. Composites of this type may achieve the desired advantageous properties (e.g. high electrical conductivity and/or optical transparency) while still having some of the physical properties of the polymer component (e.g. flexibility, processability etc.).

In the present composites, the polymer component is not particularly limited and may be selected depending on the physical characteristics that are generally desirable for the particular application. In preferred compositions the polymer is one that is soluble in a halogenated, e.g. chlorinated solvent.
Non limitative examples of halogenated solvents include chloroform, dichloromethane, tetrachloroethane, chloromethane, tetrachloromethane, bromomethane.

In preferred aspects, the polymer component is one that is soluble in halogenated solvents. For example polymers that have solubility in halogenated solvents (e.g. dichloromethane) of at least one gram of polymer per litre of solvent are preferred. Thus, suitable polymers may be selected from polyolefins, polycarbonates, polyurethanes, polythiophenes, polyanilines, unsaturated polymers, fluoropolymers, polyketones, polyaryletherketones, polypyrroles, polyamides, polyimides, polysulfones, silicones, polyesters, polyethers, polythioethers, polyaminoacids, ionomers, copolymers, blends and mixtures thereof.

Polyolefins include, in a non-limitative way, high density polyethylene, linear low density polyethylene, low density polyethylene, polyethylene acrylates, poly(methyl methacrylate), poly(vinyl alcohol), polyacrylic acid, polyacrylate, polyacrylonitrile, polybutylene, polybutylene adipate, polystyrene, polystyrene brominated, polyethylacrylate, polyethylene, polymethylpentene, polypropylene, polyvinyl acetate, polyvinyl nitrate, polyvinyl chloride.

Polycarbonates include, in a non-limitative way, aromatic-aliphatic polycarbonates, aromatic polycarbonates, aliphatic polycarbonates.

Polyurethanes include, in a non-limitative way, aromatic-aliphatic polyurethanes, aromatic polyurethanes, aliphatic polyurethanes.

Polythiophenes include, in a non-limitative way, Poly(3,4-ethylenedioxythiophene), Poly(3,4-ethylenedioxythiophene)-tetramethacrylate.

Polyanilines include, in a non limitative way, leucoemeraldine, emeraldine salt, emeraldine base, (per)nigraniline.

Unsaturated polymers include, in a non limitative way, polybutadiene, trans-polyacetylene, cis-polyacetylene.

Fluoropolymers include, in a non-limitative way, polytetrafluoroethylene and its derivatives.

Polyketones and polyaryletherketones include, in a non-limitative way, aromatic-aliphatic polyketones, aromatic polyketones, aliphatic polyketones, polyether ether ketone,

Polypyrroles include, in a non-limitative way, aromatic-aliphatic, aromatic and aliphatic derivatives.

Polyamides include, in a non-limitative way, aromatic-aliphatic, aromatic and aliphatic derivatives, polyamide 46, polyamide 6, polyamide 66, polyamide 610, polyamide 612, polyamide 10, polyamide 11, polyamide 12, polyamide 6-6T, polyamide 6-61, polyamide 6T-6I.

Polyimides include, in a non-limitative way, poly-oxydiphenylene-pyromellitimide.

Polysulfones include, in a non-limitative way, polyphenylsulfone, polyethersulfone.

Silicones include, in a non-limitative way, polydimethylsiloxane, aromatic-aliphatic polysiloxanes, aromatic polysiloxanes.

Polyesters include, in a non-limitative way, poly(butylene succinate), poly(trimethylene terephthalate), polybutylene terephthalate, polyethylene terephthalate glycol, polyethylene terephthalate, polyhydroxybutyrate, polyhydroxyvalerate, polylactic acid.

Polyethers include, in a non-limitative way, polyoxymethylene, polyphenylene oxide.

Polythioethers include, in a non-limitative way, polyphenylene sulphide.

Polyaminoacids include, in a non-limitative way, polyaspartic acid.

Ionomers include, in a non-limitative way, ethylene acrylic acid sodium ionomer, poly(styrenesulfonate).

Copolymers, blends and mixtures include, in a non-limitative way, PEDOT:PSS, acrylonitrile-butadiene-styrene copolymer, acrylic copolymers, acrylonitrile-styrene-acrylate copolymer, cyclic olefin copolymers, cyclic olefin polymers, ethylene acrylic ester copolymer, ethylene acrylic acid copolymer, ethylene epoxyacrylate copolymer, ethylene ethylacrylate copolymer, ethylene vinyl acetate copolymer, poly(butylene adipate-co-terephthalate), poly(ether-block-amides), poly(ethylene-co-methacrylic acid), methyl methacrylate copolymers, methacrylate copolymers, styrene acrylonitrile copolymer, polyvinyl chloride acetate.

Preferably the polymer is selected from polymethylmethacrylate, polystyrene, styrene-butadiene block copolymer, bisphenol-A polycarbonate, acrylonitrile-butadiene-styrene copolymer, acrylic copolymers, acrylonitrile-styrene-acrylate copolymer, cyclic olefin copolymers, cyclic olefin polymers, ethylene acrylic ester copolymer, ethylene acrylic acid copolymer, ethylene acrylic acid sodium ionomer, ethylene epoxyacrylate copolymer, ethylene ethylacrylate copolymer, ethylene vinyl acetate copolymer, high density polyethylene, linear low density polyethylene, low density polyethylene, poly ethylene acrylates, poly(butylene adipate-co-terephthalate), poly(butylene succinate), poly(ether-block-amides), poly(ethylene-co-methacrylic acid),poly(methyl methacrylate), methyl methacrylate copolymers, methacrylate copolymers, poly(trimethylene terephthalate), poly(vinyl alcohol), polyacetic acid, polyacrylic acid, polyacrylate, polyacrylonitrile, polyamide, PA 46, 6, 66, 610, 612, 10, 11, 12, 6T, 6-6T, 6-61, 6T-6I polyaspartic acid, polybutadiene, polybutylene, polybutylene adipate, polybutylene terephthalate , polycarbonate, aromatic-aliphatic polycarbonates, aromatic polycarbonates, aliphatic polycarbonates, polydimethylsiloxane, polystyrene brominated, polysulfone, polyphenylsulfone, polyethersulfone polyethylacrylate, polyethylene, polyethylene terephthalate glycol, polyethylene terephthalate, polyhydroxybutyrate, polyhydroxyvalerate, polylactic acid, polymethylpentene, polyoxymethylene acetal polymer, polyphenylene oxide, polyphenylene sulfide, polyphenylsulfone, polypropylene, polystyrene, polysulphone, polytetrafluoroethylene, polyurethane, polyhydroxybutyrate, styrene acrylonitrile, PEDOT, polypyrrole, polyaniline, polyacetylenes, mixtures, blends and co-polymers thereof.

In most preferred compositions, the carbonaceous material is multi-wall carbon nanotubes (MWNTs) and the polymer is selected from polymethylmethacrylate, polystyrene, styrene-butadiene block copolymer, and bisphenol-A polycarbonate.

The use of the bis-salphen compounds as described herein in the compositions of the present proposals, particularly in compositions in which the carbonaceous material is CNTs, results in good dispersion of the carbonaceous material in the polymer component and reduces the spontaneous agglomeration and clumping of the carbonaceous material (especially CNT material) in the polymer. This reduction in the agglomeration is advantageous because any agglomeration can lead to impaired optical, mechanical and conductive properties. The presence of a bis-salphen compound as described herein in compositions comprising CNTs (in particular MWNTs) results in the CNTs dispersing homogeneously with a unimodal aggregate size distribution profile which closely matched the diameter of the CNTs used, e.g. a size distribution profile from about 10 nm to about 1 µm, particularly 30 to 750 nm, more particularly 100 to 250 nm.

Furthermore, the use of the bis-salphen compounds as described herein in compositions in which the carbonaceous component is CNTs results in networks of CNTs, in which the CNTs are in extended electrical contact, being formed (e.g. by self-assembly) in the dispersion medium (e.g. polymer matrix, surface).

In particular aspects, the polymer-comprising compositions contain up to about 1 wt.% of the bis-salphen compound, more particularly up to 0.5 wt.%, most particularly up to about 0.1 wt.%. If too much of the bis-salphen compound is included, the cost of the compositions will increase making the compositions less commercially viable, and some physical properties of the polymer may be lost. The lower amount of bis-salphen compound in the composition is not particularly limited. However, below about 0.001 wt.%, the advantageous effects of the bis-salphen compounds may decrease.

In particular aspects, the polymer-comprising compositions contain about less than about 30 wt.%, preferably less than 15 wt.%, preferably less than 10 wt.%, more preferably less than 1 wt.%, most preferably less than 0.5 wt.% of the carbonaceous material. As a lower limit, the polymer-comprising compositions preferably contain more than about 0.000001 wt.%, preferably more than 0.00001 wt.%, more preferably more than 0.0001 wt.%, most preferably more than 0.01 wt.% of the carbonaceous material.

In particular aspects, the polymer-comprising compositions contain about 0.00001 wt.% to about 30 wt.% of the carbonaceous material (e.g. CNTs), preferably 0.0001 wt.% to 10 wt.%, most preferably 0.01 wt.% to 5 wt.%.

If the amount of carbonaceous material is too low, the desired physical properties (e.g. high electrical conductivity) of the composition may not be observed. If the amount of carbonaceous material is too high, the optical film transparency of the composition may be degraded.

The amount of polymer or gel component (when present) in the composition is preferably greater than 50 wt.%, more preferably greater than 60 wt.%, more preferably greater than 70 wt.%, more preferably greater than 80 wt.%, even more preferably greater than 90 or 95 wt.% of the total weight of the composition.

The present proposals also relate to a method for preparing a composition as described herein. The method comprises the steps of: preparing a mixture of carbonaceous material with a compound as described herein in a solvent; and eliminating the solvent to provide the composition. The mixture may further incorporate a polymer or gel component as defined herein.

In some embodiments, the solvent used in the method for preparing a composition as described herein is a halogenated solvent. Preferably, the solvent used in the method for preparing a composition as described herein is selected from chloroform, dichloromethane, and tetrachloroethane; more preferably, the solvent is dichloromethane.

Preferred methods comprise the steps of: preparing a mixture of carbonaceous material with a compound as described herein in a solvent; and eliminating the solvent to provide the composition; wherein the concentration of the compound as described herein of the mixture used in the first step is from 10⁻⁹ M to 10⁻³ M; preferably the concentration of the compound as described herein of the mixture used in the first step is from 10⁻⁶ to 10⁻⁴ M. More preferably, the concentration of the compound as described herein of the mixture used in the first step is 10⁻⁵ M.

In some methods, a solution of the polymer component and the bis-salphen compound in the solvent may be prepared first and, separately, a solution or suspension of the carbonaceous component may be prepared in a solvent (preferably the same solvent as used for the polymer and bis-salphen solution), after which the two solutions/suspensions are combined and mixed (optionally with sonication e.g. using an ultrasound probe or bath, to improve mixing), before elimination of the solvent(s) to provide the composition.

In some methods, a solution of the polymer component in the solvent may be prepared first and, separately, a solution or suspension of the carbonaceous component with the bis-salphen compound may be prepared in a solvent (preferably the same solvent as used for the polymer solution), after which the two solutions/suspensions are combined and mixed (optionally with sonication e.g. using an ultrasound probe or bath, to improve mixing), before elimination of the solvent(s) to provide the composition.

In some methods, a solution of the polymer component in the solvent may be prepared first and, separately, a solution or suspension of the carbonaceous component may be prepared in a solvent (preferably the same solvent as used for the polymer solution), and, separately a solution of the bis-salphen compound may be prepared in a solvent (preferably the same solvent as used for the polymer solution) after which the three solutions/suspensions are combined and mixed (optionally with sonication e.g. using an ultrasound probe or bath, to improve mixing), before elimination of the solvent(s) to provide the composition.

In some methods, a solution or suspension of the polymer component and the carbonaceous component in the solvent may be prepared first and, separately, a solution of the bis-salphen compound may be prepared in a solvent (preferably the same solvent as used for the polymer and carbonaceous component solution), after which the two solutions/suspensions are combined and mixed (optionally with sonication e.g. using an ultrasound probe or bath, to improve mixing), before elimination of the solvent(s) to provide the composition.

In preferred methods, the solvent and the polymer components are selected such that the polymer is soluble in the solvent. This allows intimate mixing of the polymer and carbonaceous material and an even dispersion of the carbonaceous material throughout the polymer matrix.

In preferred methods, the solvent is a halogenated solvent, preferably a chlorinated solvent, more preferably a solvent selected from dichloromethane, chloroform, and tetrachloroethane. Even more preferably, the solvent is dichloromethane.

The step of eliminating the solvent may comprise formation of the composition by simple evaporation, dropcasting, spin coating, doctor-blade film formation, or compression moulding, during which the solvent is eliminated to leave the product composition.

The methods described herein may further comprise a step of processing the composition to shape or form it into a desired component. For example the method may further comprise the step of melt extrusion, or injection moulding, after elimination of the solvent.

The present invention also covers all possible combinations of particular and preferred embodiments, options and preferences described herein.

Components, composites and compositions formed by methods described herein also form part of the present proposals.

In the usual way, in the numerical ranges described herein, the upper and lower limits of the ranges are distinct proposals.

### EXAMPLES

The present proposals are illustrated in the following examples which are provided for illustrative purposes only and are not intended to limit the scope of the invention.

### Characterization Techniques

Generally, characterization of synthesised compounds was by elemental analysis, Matrix-assisted laser desorption/ionization-Time of flight (MALDI-TOF) and High Resolution Mass Spectrometry, ¹H and ¹³C Nuclear Magnetic Resonance (¹H NMR and ¹³C NMR), ¹³C{¹H} Distortionless Enhancement by Polarization Transfer (DEPT) and X-ray crystallography molecular structure. All NMR measurements were carried out on a Bruker-400 MHz spectrometer at ambient temperature unless stated otherwise, and chemical shifts are given in parts per million versus TMS. Mass spectrometric data were obtained from the Research Support Unit of the ICIQ and MALDI-TOF experiments were carried out with pyrene as matrix.

### Transmission Electron Microscopy (TEM) Analysis

TEM analysis was performed on a JEOL model 1011 electron microscope at 100 kV. Liquid samples analyzed by TEM were prepared by dropcasting 0.35 mM solutions of the corresponding compound (previously dissolved in the selected solvent) on formvar carbon film-covered square mesh copper grids and dried completely for 4 h. Solid film samples were embedded in a standard preparative epoxy-based resin, cured for 72 h at 80 °C, cut with a microtome and fixed on a double copper grid fixation system for analysis.

### Environmental Scanning Electron Microscopy (ESEM)

Microscopic analysis was carried out at 4 Torr, 10 °C and 30 kV using a FEI Quanta microscope with Everhart-Thornley Detector (ETD), model 600 coupled to an X-ray microanalyzer. Liquid samples for ESEM analysis were prepared by dropcasting required solutions (0.35 mM) on a metal slide for wet sampling and completely evaporated prior to analysis. Solid samples were fixed on sample holders without any previous treatment and directly analyzed at the microscope.

### Electrical Characterization of Materials

Surface electrical resistivity of solid films was determined with a Keithley Model 8009 high resistivity test fixture with integrated Faraday box electrostatic shielding system coupled to a Keithley 6517A electrometer. Film samples with the dimension 6 cm x 6 cm were directly analyzed within the fixture without any previous treatment at applied voltages of 100 V and 500 V. Volume electrical resistivity measurements were carried out under the same conditions.

### Optical characterization of materials

UV-vis spectra and optical transmission measurements were recorded on a Shimadzu UV1800 Spectrophotometer. UV-Vis-NIR measurements were carried out on a Lamba 1050 PerkinElmer spectrophotometer equipped with a PMT, InGaAs and PbS detectors system, double beam optics, double monochromator and D2 and W light sources.

### Materials

All starting materials were purchased from commercial sources and used without further purification.

### Bisphenol A polycarbonate

The polymers used in the following examples are bis phenol A polycarbonate purchased from Bayer Material Sciences with catalogue numbers
MAKROLON OD2015 (used Examples 2-10) and MAKROLON ML3105 (used in Example 11).

### Unmodified Carbon Nanotubes

The multiwall carbon nanotubes (MWNTs) used in the experiments - >99% purity, >>1 µm length and 13-16 nm outer mean diameter (used in Examples 6, 7, 11), or >95% purity, 100-700 nm length and 15-42 outer mean diameter (used in Examples 1, 5, 8, 10), or >99% purity, >>1 µm length and 8-15 nm outer mean diameter (used Examples 2,3,4,9) - were purchased from Bayer Technology Services (Leverkusen, Germany), Nanocyl SA (Sambreville, Belgium), and HeJi (Shenzhen, China) respectively. The carbon nanotubes were dried before use at 120 °C, under vacuum (300 mBar) for 90 min and stored under dry and low-pressure conditions until needed.

### Carboxylated Carbon Nanotubes

Multiwall carbon nanotubes were refluxed in 2.6 M nitric acid for 4 h to oxidize the metallic impurities remaining from the synthesis (C.A. Furtado, U.J. Kim, H.R. Gutierrez, L. Pan, E.C. Dickey, P.C.J. Eklund, J. Am. Chem. Soc. 2004, 126, 6095). The carbon nanotubes became carboxylated at the ends after this oxidation step (MWNT-COOH). The MWNT-COOH in nitric acid solution were filtered and thoroughly rinsed with water to remove the acid completely. The filtered MWNT-COOH were dried overnight at 80 °C.

The following compounds were prepared using previously reported methodologies:
(E)-2-(((2-aminophenyl)imino) (phenyl)methyl) phenol (A. Kämpfe, E. Kroke and J. Wagler. Eur. J. Inorg. Chem. 2009, 1027-1035);
3,3'-diformyl-2,2'-dihydroxy-1,1'-biphenyl (H.-C. Zhang, W.-S. Huang, L. Pu, J. Org. Chem. 2001, 66, 481-487);
2-hydroxy-[1,1'-biphenyl]-3-carbaldehyde (C. Görl, H. G. Alt, J. Organomet. Chem. 2007, 692, 5727-5753);
3,3'-bis((E)-((2-((E)-((2-hydroxyphenyl)(phenyl)-methylene)amino)phenyl)imino)methyl)-[1,1'-biphenyl]-2,2'-diol (bis-salphen ligand **1**), and di-Zn-salphen complex **1a** (M. V. Escárcega-Bobadilla, G. Salassa, M. Martínez Belmonte, E. C. Escudero-Adán and A. W. Kleij. Chem.-Eur. J. 2012, 18, 6805-6810); and
di-Ni-salphen complex 1 b (Escárcega-Bobadilla M.V., Zelada-Guillén G.A., Pyrlin S., Wegrzyn M., Ramos M.M.D., Giménez E., Stewart A., Maier G., Kleij A.W. in preparation).

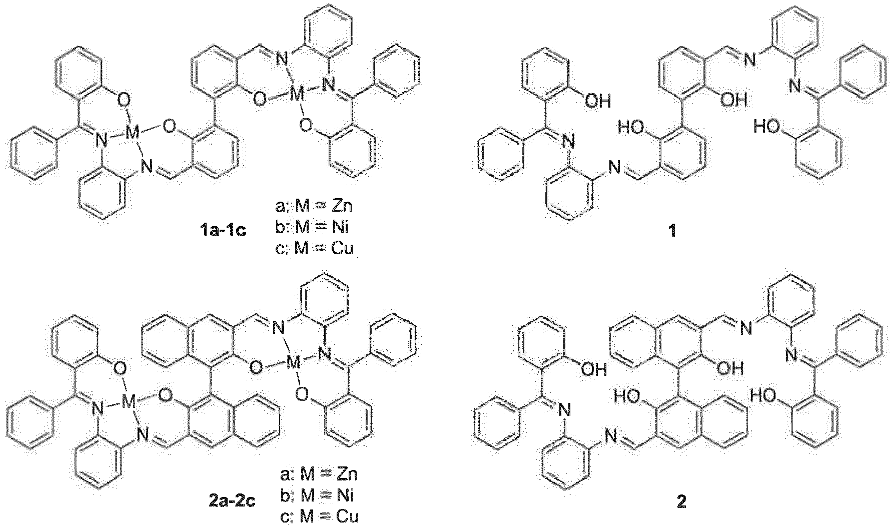

### Preparative Example 1 - Di-Cu-Salphen 1c

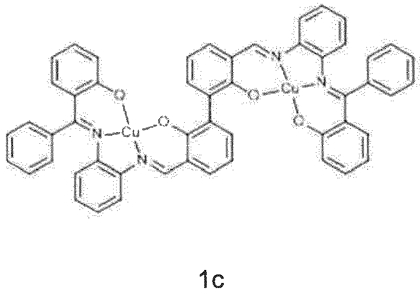

A solution of 1a (50 mg, 0.06 mmol) in 10 mL of THF and Cu(AcO)₂ (21 mg, 0.12 mmol) in 5 mL of MeOH was stirred for 18h. The brownish suspension was filtered off and the solid washed with MeOH. Brown solid, 54 mg, 99%.

MS (MALDI+) m/z 904.2 ([M+]). Elemental analysis calculated (%) for C₅₂H₃₄Cu₂N₄O₄·2H₂O C 66.30, H 4.07, N 5.95 found C 66.58, H 4.00, N 5.95.

### Preparative Example 2 - (S)-3,3'-bis((E)-((2-((E)-((2-hydroxy-phenyl)(phenyl)methylene)amino)phenyl)imino)methyl)-[1,1'-bina phthalene]-2,2'-diol ligand 2

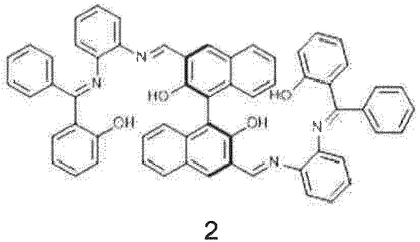

(*E*)-2-(((2-aminopheny))imino)(phenyl)methyl)phenol (200 mg, 0.7 mmol) in 2 mL of CHCl₃ and (S)-2,2'-dihydroxy-[1,1'-binaphthalene]-3,3'-dicarbal-dehyde (119 mg, 0.35 mmol) in 5 mL of MeOH were refluxed for 24 h. The precipitate formed was then filtered off. Yellow solid, 300 mg, 97%.

HRMS (TOF ES⁺) *m*/*z* 883.3326 ([M⁺H]) C₆₀H₄₃N₄O₄ requires 883.3284.

### Preparative Example 3 - Di-Zn-Salphen 2a

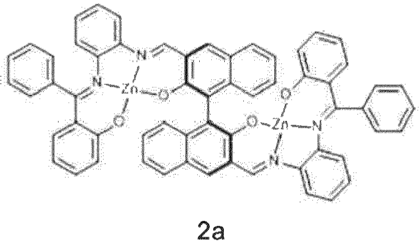

To a solution of ligand 2 (100 mg, 0.13 mmol) in 6 mL of anhydrous THF, ZnEt₂ (0.29 mL 1 M in hexanes, 0.29 mmol) was slowly added. The reaction was stirred at room temperature 18 h. The red precipitate was filtered off. Red solid, 127 mg, 97%.

MS (MALDI+) m/z 1009.1 ([M⁺H]). Elemental analysis calculated (%) for C₆₀H₃₈N₄O₄Zn₂·14H₂O C 56.30, H 5.35, N 4.38 found C 56.02, H 3.74, N 4.91.

### Other Salphen compounds

Di-metallic-Salphen compounds can be prepared from di-, tri-, or tetravalent transition metal salts, depending on the metal center required, and using the ligands 1 or 2 in an analogous procedure to the preparation of Di-Cu-Salphen 1c from Di-Zn-Salphen 1 a. For instance, using Ni(OAc)₂ instead of Cu(OAc)₂ in the preparative example 1, compound 1 b was isolated in 98% yield as a brown solid.

### Example 1 - Dispersion of nanotubes within dichloromethane using compounds 1 a or 2a

Table 1 summarizes the aggregate-size distribution profile, as recorded by dynamic light scattering (DLS), of:
(i) DCM solutions of the complexes 1a or 2a at the indicated concentration;
(ii) DCM suspensions of MWNTs (length: 100-700 nm, external diameter: 15-42 nm) at the indicated concentration; and
(iii) mixtures of the complexes 1 a, or 2a with the MWNTs in DCM at the indicated concentration.

**Table 1**

| [1a] (nM) | [2a] (nM) | [MWNT]( mg/mL) | Peak 1 diameter value (nm) | Peak 2 diameter value (nm) | Peak 3 diameter value (nm) |
|---|---|---|---|---|---|
| 0 | 0 | 0.02 | 164 | 531 | 5560 |
| 70 | 0 | 0 | 2.3 | 190 | 5560 |
| 0 | 70 | 0 | 0.6 | 6.5 | 164 |
| 70 | 0 | 0.02 | 220 | - | - |
| 0 | 70 | 0.02 | 38 | - | - |
| 280 | 0 | 0.08 | 615 | - | - |
| 0 | 280 | 0.08 | 122 | - | - |

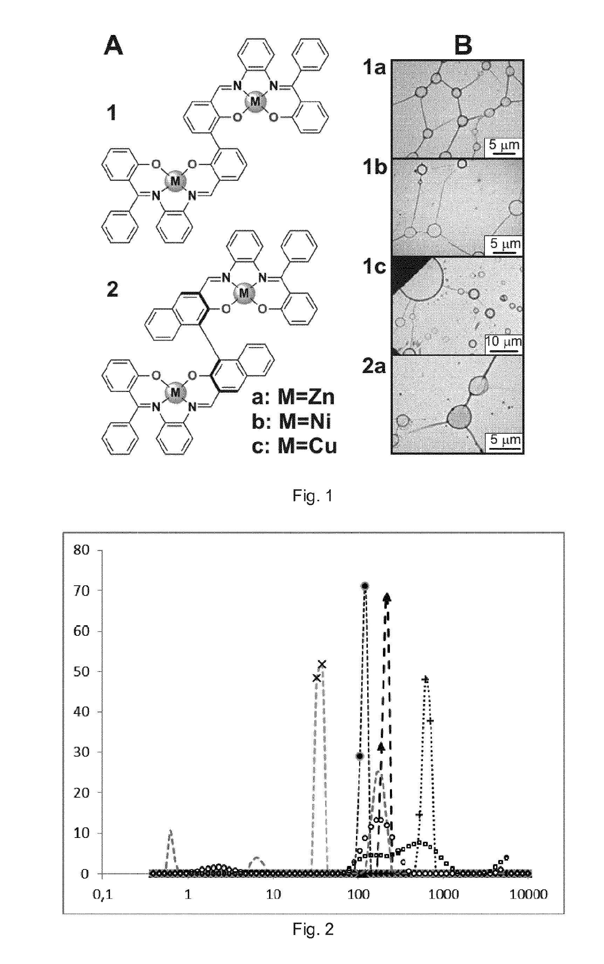

Table 1 shows that it is possible to avoid the spontaneous agglomeration of MWNTs in DCM dispersions by incorporating one of the complexes 1 a or 2a. The presence of the complex facilitates the nanotubes to homogeneously disperse into an unimodal aggregate size distribution profile. Figure 2 shows the DLS profile of the prepared materials. The presence of single sharp peaks in the DCM profiles for samples containing both MWNTs and compound 1a or 2a indicates good dispersion of the MWNTs. The correlation between the average diameter of the MWNTs used in the samples and the DCM values at which the peaks for the mixtures with compounds 1 a and 2a as observed indicates that the MWNTs are close to monodisperse.

Figure 3 shows a TEM image of a drop-cast solution of 1 a (70 µM) containing MWNT (0.02 g/mL), wherein the carbon nanotubes and complex 1 a are organized into an extended network. Interestingly, the presence of the bis-salphen compound or complex facilitates the nanotubes to homogeneously disperse into a unimodal aggregate size distribution profile ranging from 105 to 220 nm, depending on the complex used. These values are closely similar to the size dimensions of the nanotubes used, which indicates the predominance of individually dispersed nanotubes in solution.

The results show that a compound of formula (I) or a coordination complex thereof is useful for the dispersion of a carbonaceous material, such as carbon nanotubes into halogenated solvents, by the formation of unique structures.

### Example 2 - General method for obtaining preparative solutions of composite

Preparative solutions of composite materials of different compositions containing bisphenol A polycarbonate, compound 1 a, 2a or 1 c and MWNT, were prepared following the steps:
(i) The different following solutions were prepared:
   a) Solution 1: A solution of 1 a, 2a or 1 c in dichloromethane (DCM) at a concentration of 0.7 mM.
   b) Solution 2: Solutions of polymers were prepared prior to material processing by dissolving bisphenol A polycarbonate MAKROLON OD2015 in anhydrous dichloromethane (DCM, 99.8%) at a concentration of 0.1 g per mL.
   c) Solution 3: Solution 2 and solution 1 were mixed in a 9:1 volume ratio, so that the concentration of solution 3 in compound 1 a, 1 c or 2a is 70 µM.
   e) Dispersion 1: Separately, liquid dispersions of carbon nanotubes in DCM (either unmodified or carboxylated carbon nanotubes) were prepared and sonicated for 30 minutes at 25 °C in a standard bath sonicating system. The amount of MWNT in the suspension varies depending on the desired load of MWNT in the final composite material.
   f) Solution 4: Liquid mixtures of solution 3 and dispersion 1 were prepared by mixing 10 volume parts of solution 3 with one volume part of dispersion 1.

Comparative preparative solutions, i.e. solutions that do not contain MWNT and/or do not contain compound 1 a, 1 c or 2a, were also prepared following the same procedure but using neat DCM instead of solution 1 and/or dispersion 1.

According to this procedure, the following amounts of MWNT have been used:

| Amount of MWNT in dispersion 1 (g per litre) | Masterbatch composite load in MWNT (wt.%) |
|---|---|
| 47.4 | 5 |
| 37.5 | 4 |
| 27.9 | 3 |
| 10.4 | 2 |
| 9.1 | 1 |
| 2.0 | 0.22 |
| 1.0 | 0.11 |
| 0.2 | 0.022 |
| 0.1 | 0.011 |
| 0.02 | 0.0022 |
| 0.01 | 0.0011 |
| 0.002 | 0.00022 |
| 0.001 | 0.00011 |

### Example 3 - General method for obtaining films by Doctor Blade Technique

Films were prepared by the Doctor Blade technique from casting the preparative solutions 4 of example 2 at the desired nanotubes:(salphen + polymer) mass ratio under high-efficiency particulate air-filtered laminar flow conditions. An aliquot of the solution (commonly 1 - 5 mL, depending on the surface to cover and the film thickness desired) was deposited on a polyethylene terephthalate sheet used as support and the cast solution was homogeneously spread onto the support at a spreading speed ranging from 2.5 mm/s to 30 mm/s at 25 °C by means of a standardized iron blade with sub-micrometer-sized adjusting capabilities. The film therein formed was dried at low vacuum (10-100 mBar) and 25 °C for 1-2 h. Films of variable thicknesses are possible by adjusting the blade height (commonly between 0.05 µm and 500 µm). Blank films were prepared from liquid mixtures of nanotubes:polymer in variable mass ratios between 0:100 and 5:95. Salphen-containing films were prepared from liquid mixtures of nanotubes:(salphen + polymer) in variable mass ratios between 0:100 and 5:95; the load of salphen in the salphen + polymer solution was adjusted by selecting the concentration range described in Example 2 above.

### Example 4 - General method for obtaining films by Spin Coating Technique

Films were prepared by spin coating from casting the preparative solutions of Example 2 at the desired nanotubes:(salphen + polymer) mass ratio under a closed injection system. An aliquot of the solution (commonly 1-10 mL, depending on the film thickness desired) was deposited on a circular flat aluminium container (6 cm diameter) adapted to a spin coater. The solution was then dispersed by spinning at 10-100 rpm (preferably 60 rpm) and dried at 25 °C and 1 Atm. The film therein formed was dried at low vacuum (10-100 mBar) and 25 °C for 1-2 h and separated from the aluminium container. Thickness of the films was controlled during preparation procedure to a final value of approximately 10, 50 or 100 µm in the solid state. Blank films were prepared from liquid mixtures of nanotubes:polymer in variable mass ratios between 0:100 and 5:95. Salphen-containing films were prepared from liquid mixtures of nanotubes:(salphen + polymer) in variable mass ratios between 0:100 and 5:95; the load of salphen in the salphen + polymer solution was adjusted by selecting the concentration range described in the Example 2 above.

### Example 5 - General method for obtaining films by Compression moulding

Films were prepared by compression moulding from casting the preparative solutions of Example 2 at the desired nanotubes:(salphen + polymer) mass ratio. An aliquot of the solution (commonly 1 - 10 mL, depending on the surface to cover and the film thickness desired) was deposited on a flat compressing surface protected by a disposable aluminium layer. The cast solution was homogeneously spread onto the compressing surface and dried at 25 °C and 1 Atm until complete evaporation was achieved. The material therein prepared was finally moulded melting at 300 °C and compression at 10⁴ Pa. Blank films were prepared from liquid mixtures of nanotubes:polymer in variable mass ratios between 0:100 and 5:95. Salphen-containing films were prepared from liquid mixtures of nanotubes:(salphen + polymer) in variable mass ratios between 0:100 and 5:95; the load of salphen in the salphen+polymer solution was adjusted by selecting the concentration range described in Example 2 above.

### Example 6 - General method for obtaining parts by Melt Extrusion

Parts were prepared by melt extrusion starting from the preparative solutions of Example 2 at the desired nanotubes:(salphen + polymer) mass ratio. Salphen-containing extruded parts were prepared from liquid mixtures of nanotubes:(salphen + polymer) in variable mass ratios between 0:100 and 5:95; the load of salphen in the salphen + polymer solution was adjusted by selecting the concentration range described in Example 1 above. The liquid mixtures of nanotubes:(salphen + polymer) were completely evaporated at low vacuum 10-200 mBar, 40 °C and stirring conditions (20-80 rpm). The solid material was recovered and ground to a grain size of approximately 400 µm - 4 mm. The solid material was fed into a standard extruder for thermoplastics (MiniLab Rheomex CTW5. HAAKE, Germany). The extrusion process was carried out at the following conditions: Tm = 280 °C, Ts = 280 °C, moment = 18 N cm and n = 60 min⁻¹. The form of the extruded parts was adjusted by commercially available standard nozzles.

### Example 7 - General method for obtaining parts by Injection moulding

Parts were prepared by melt extrusion starting from the preparative solutions of Example 2 at the desired nanotubes:(salphen + polymer) mass ratio. Salphen-containing moulded parts were prepared from liquid mixtures of nanotubes:(salphen + polymer) in variable mass ratios between 0:100 and 5:95; the load of salphen in the salphen + polymer solution was adjusted by selecting the concentration range described in Example 1 above. The liquid mixtures of nanotubes:(salphen + polymer) were completely evaporated at low vacuum 10-200 mBar, 40 °C and stirring conditions (20-80 rpm). The solid material was recovered and ground to a grain size of approximately 400 µm - 4 mm. The solid material was fed into a standard injection moulding for thermoplastics (Injection moulding device model MiniJet II, Thermo Scientific, Germany). The injection moulding experiments were carried out at the following conditions: Cylinder Temperature = 280 °C, Mould Temperature = 105 °C, Pressure = 500 Bar, Holding Pressure = 300 Bar, Time = 15 s. The form, height, length and width of the moulded parts was adjusted by commercially available standard hardened steel moulds.

### Example 8 - Dispersion of nanotubes within a polymer matrix using compound 2a - Composite films

A blank bisphenol A polycarbonate film was prepared from a cast solution of polymer in DCM by compression moulding as described in Example 5. A composite film was similarly prepared with the complex 2a (0.07 %-wt.) and MWNTs (0.02 %-wt). Environmental scanning electron microscopy (ESEM) was performed on each sample and a superficial view is shown in Fig. 4 (part A showing the blank polycarbonate and part B showing the composite including the complex 2a and the MWNTs).

Similarly, films were prepared by casting (spin-coating) from the preparative solutions of Example 2:
- solution of bisphenol A (BPA) polycarbonate in DCM;
- solution of BPA polycarbonate with complex 1 a 0.07 wt.% in DCM; and
- suspension of BPA polycarbonate with complex 1 a 0.07 wt.% and MWNTs 0.02 wt.%

Transversal cuts observed by TEM are shown in Fig. 5 (part A showing the polycarbonate alone; part B showing the polycarbonate & complex 1 a; and part C showing the polycarbonate, complex 1 a & MWNTs).

Figures 4 and 5 show that the compound of formula (I) or a coordination complex thereof allows for dispersing the nanotubes within a polymer matrix. Various film formation techniques have been used and self-assembled structures are maintained.

### Example 9 - Resistivity measurements of composite films

Different composite film compositions were prepared by spin-coating of the preparative solutions of Example 2 having the concentrations specified in Table 2 and Table 3. In all cases, the composite film comprises 0.07 % by weight of compound of formula (I) (i.e. 1 a, 1 c, or 2a). In Tables 2 and 3, "blank" refer to composite films prepared from bis phenol-A (BPA) polycarbonate and carbon nanotubes, i.e. free of the compound of formula (I). The typical morphological characteristics of the films thus obtained are: diameter: 6.5 cm; nominal thickness: 0.1 mm; average film density: 1.6 g/cm³. Surface and volume resistivity was measured as described above.

**Table 2**

| **Compound (I)** | **% wt MWNT** | **Surface resistivity (ohm)** |
|---|---|---|
| Blank | 5 | 190000 |
| Blank | 3 | 4,05378E+12 |
| Blank | 1 | 6,02239E+14 |
| Blank | 0,22 | 1,34697E+14 |
| Blank | 0,11 | 2,04107E+14 |
| Blank | 0,022 | 2,17182E+14 |
| Blank | 0,011 | 2,95477E+14 |
| Blank | 0,0022 | 3,11397E+14 |
| Blank | 0,0011 | 3,63556E+14 |
| Blank | 0,00022 | 4,39481E+14 |
| Blank | 0,00011 | 6,13302E+14 |
| Blank | 0 | 5,23139E+16 |
| 1c | 0,22 | 3,18573E+12 |
| 1c | 0,022 | 2,98494E+14 |
| 1c | 0,0022 | 3,4785E+14 |
| 1c | 0,00022 | 6,61377E+14 |
| 1c | 0 | 1,07956E+15 |
| 1a | 5 | 46000 |
| 1a | 3 | 386524,8667 |
| 1a | 0,22 | 5,16024E+13 |
| 1a | 0,11 | 2,09714E+14 |
| 1a | 0,022 | 2,42327E+14 |
| 1a | 0,011 | 2,56274E+14 |
| 1a | 0,0022 | 4,01555E+14 |
| 1a | 0,0011 | 5,81021E+14 |
| 1a | 0,00022 | 9,14042E+14 |
| 1a | 0,00011 | 1,70782E+15 |
| 1a | 0 | 7,11571E+16 |
| 2a | 5 | 40650 |
| 2a | 3 | 247677,5 |
| 2a | 2 | 573318,4 |
| 2a | 1 | 295980915 |
| 2a | 0,22 | 4,25928E+13 |
| 2a | 0,11 | 1,21096E+14 |
| 2a | 0,022 | 1,34371E+14 |
| 2a | 0,011 | 3,72199E+14 |
| 2a | 0,0022 | 2,65073E+14 |
| 2a | 0,0011 | 3,7314E+14 |
| 2a | 0,00022 | 3,78432E+14 |
| 2a | 0,00011 | 6,69138E+14 |
| 2a | 0 | 4,2438E+16 |

Fig. 6 shows the surface resistivity (ohm) measurements from Table 2 of polycarbonate films with different compositions.
X axis: load of MWNT in weight % (linear scale)
Y axis: Surface resistivity (ohm), logarithmic scale

**Table 3**

| **Compound (I)** | **% wt MWNT** | **Volume resistivity (ohm·cm)** |
|---|---|---|
| Blank | 5 | 778,6666667 |
| Blank | 4 | 21360 |
| Blank | 2 | 9226,666667 |
| 1c | 5 | 77,86666667 |
| 1c | 2 | 3240 |
| 1a | 5 | 24 |
| 1a | 4 | 318,4 |
| 1a | 3 | 489,63808 |
| 1a | 2 | 5920 |
| 2a | 5 | 392 |
| 2a | 3 | 4221,792533 |
| 2a | 2 | 7760 |

Fig. 7 shows volumetric resistivity (ohm·cm) of different films of composite materials from Table 3 containing 0.07 wt.% bis-salphen compound: Film disk dimensions: 33.2 cm² (6.5 cm diameter), 100 µm thickness, Average film density: 1.6 g/cm³, Average mass/film disk: 540 mg, Average optical transmittance: 60-90%, depending on the type of sample and thickness X axis: load of MWNT in weight % (logarithmic scale)
Y axis: Volumetric resistivity (ohm·cm), logarithmic scale

Tables 2 and 3 along with Figs 6 and 7 show that composite materials comprising the compound of formula (I) or a coordination complex thereof have a lower electrical percolation threshold than composite materials formed from BPA polycarbonate and MWNT alone in the same conditions. These results also show that the selection of the metals of the compound of formula (III) allows for fine-tuning the properties of the material.

Figure 8 shows the transmittance (in %) for different wavelengths (in nm) of the prepared films.

### Example 10: Composite polymer films with carboxylated nanotubes

A composite film of BPA, compound 2a (0.07 wt.%) and carboxylated multi-wall carbon nanotubes,(0.02% wt.%) was prepared by compression moulding as described above (example 5). The obtained film was studied by TEM and compared with a film similarly prepared using non-functionalized carbon nanotubes. The figure 10 shows that functionalization of the carbon nanotubes can be used to influence the assembly of the carbonaceous material within the matrix.

### Example 11: Composite polymer parts obtained by extrusion and injection moulding

Composite polymer parts of BPA polycarbonate, compound 1 a, and MWNTs having the compositions detailed in Table 4 have been prepared by extrusion and injection moulding as described in the general method above (examples 6 and 7). The obtained materials were studied by TEM as shown in Figure 9.

**Table 4**

| Sample | [1a] wt.% | [MWNT] wt.% |
|---|---|---|
| 1 | 0 | 0 |
| 2 | 0.07 | 0 |
| 3 | 0 | 0.3 |
| 4 | 0 | 3 |
| 5 | 0.07 | 0.3 |
| 6 | 0.07 | 3 |

The results of figure 9 show that the even dispersion and interconnection of the carbonaceous material is preserved within the polymer matrix, even when the material is submitted to harsh process conditions, such as melt extrusion and injection moulding.

## Claims

1. A compound of formula (I) or a coordination complex thereof with a metal ion selected from the group consisting of Ni(II), Cu(II), Sc(III), Fe(II), Fe(III), Ca(II), Co(II), Co(III), Os(III), Os(IV), AI(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II) for use as a dispersing agent for improvement of dispersion of a carbonaceous material in a dispersion medium 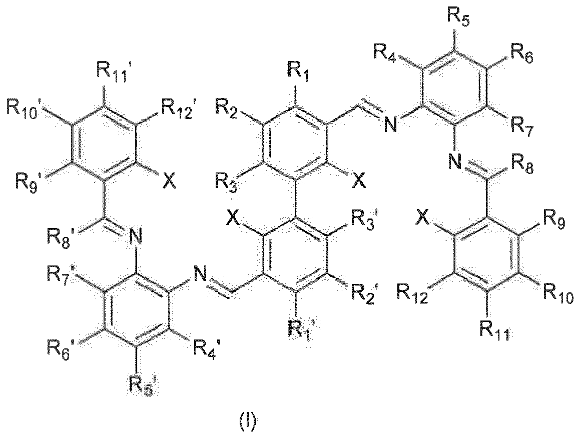 wherein:
each X is independently selected from OH and SH;
R₁, R₂, R₃, T₁' R₂' and R₃' are each independently selected from the group consisting of halogen, hydrogen, cyano and nitro; provided that one or more of the pairs of groups R₁ and R₂, R₂ and R₃, R₁' and R₂', R₂' and R₃' together with the carbon atoms to which they are attached optionally form a phenyl ring or a 5 or 6 membered heteroaromatic ring having one or more heteroatoms selected from O, N and S; wherein one or more of the hydrogen atoms of the aromatic or heteroaromatic ring is optionally replaced by a radical selected from halogen, nitro and cyano;
R₄, R₅, R₆, R₇, R₉, R₁₀, R₁₁, R₁₂, R₄', R₅', R₆', R₇', R₉', R₁₀', R₁₁', and R₁₂' are each independently selected from H, halogen, nitro, cyano, C₁₋₆ alkyl, -O(C₁₋₆ alkyl), -(C=O)C₁₋₆ alkyl, -O(C=O)C₁₋₆ alkyl, -(C=O)O(C₁₋₆ alkyl), C₃₋₈ cycloalkyl, C₃₋₈ heterocycloalkyl, phenyl, and C₅₋₆ heteroaryl, wherein any of the C₃₋₈ cycloalkyl, C₃₋₈ heterocycloalkyl, phenyl, and C₅₋₆ heteroaryl groups are optionally substituted with one or more groups selected from halogen, nitro, cyano, and C₁₋₆ alkyl; provided that one or more of the pairs of groups R₄ and R₅, R₅ and R₆, R₆ and R₇, R₉ and R₁₀, R₁₀ and R₁₁, R₁₁ and R₁₂, R₄' and R₅', R₅' and R₆', R₆'and R₇', R₉' and R₁₀', R₁₀' and R₁₁', R₁₁' and R₁₂' together with the carbon atoms to which they are attached optionally form a phenyl ring or a 5 or 6 membered heteroaromatic ring having one or more heteroatoms selected from O, N and S; wherein one or more of the hydrogen atoms of the aromatic or heteroaromatic ring is optionally replaced by a radical selected from halogen, nitro, and cyano, C₁₋₆ alkyl, C₁₋₆ haloalkyl, -O(C₁₋₆ alkyl), - (C=O)C₁₋₆ alkyl, -O(C=O)C₁₋₆ alkyl, and -(C=O)O(C₁₋₆ alkyl);
R₈ and R₈' are independently selected from a phenyl ring and a 5 or 6 membered heteroaromatic ring having one or more heteroatoms selected from O, N and S, the phenyl ring or 5 or 6 heteroaromatic ring optionally being bridged or fused with one or more 5 to 12 membered ring; wherein one or more of the hydrogen atoms of each ring is optionally replaced by a radical selected from halogen, nitro, and cyano.

2. The compound according to claim 1 wherein R₈ and R₈' are each independently selected from phenyl and a 5 or 6 membered heteroaromatic ring having one or more heteroatoms selected from O, N and S, the phenyl group or 5 or 6 heteroaromatic ring optionally being bridged or fused with one or more 5 to 7 membered ring.

3. The compound according to any of the preceding claims wherein R₈ and R₈' are each independently selected from phenyl, naphthyl, and biphenylyl.

4. The compound according to any of the preceding claims wherein R₈ and R₈' are both phenyl.

5. The compound according to any one of the preceding claims, wherein R₁, R₂, R₃, R₁', R₂', R₃' are each independently selected from the group consisting of halogen, hydrogen, cyano and nitro; provided that one or more of the pairs of groups R₁ and R₂, R₂ and R₃, R₁' and R₂', R₂' and R₃' together with the carbon atoms to which they are attached optionally form a phenyl ring or a 5 or 6 membered heteroaromatic ring having one or more heteroatoms selected from O, N and S; and
R₄, R₅, R₆, R₇, R₉, R₁₀, R₁₁, R₁₂ R₄', R₅', R₆', R₇', R₉', R₁₀', R₁₁', and R₁₂' are each independently selected from H, halogen, nitro, cyano, phenyl, and C₅₋₆ heteroaryl, wherein any of the phenyl and C₅₋₆ heteroaryl groups are optionally substituted with one or more groups selected from halogen, nitro, cyano, and C₁₋₆ alkyl; provided that one or more of the pairs of groups R₄ and R₅, R₅ and R₆, R₆ and R₇, R₉ and R₁₀, R₁₀ and R₁₁, R₁₁ and R₁₂, R₄' and R₅', R₅' and R₆', R₆' and R₇', R₉' and R₁₀', R₁₀' and R₁₁', R₁₁' and R₁₂' together with the carbon atoms to which they are attached optionally form a phenyl ring or a 5 or 6 membered heteroaromatic ring having one or more heteroatoms selected from O, N and S; wherein one or more of the hydrogen atoms of the aromatic or heteroaromatic ring is optionally replaced by a radical selected from halogen, nitro, and cyano.

6. The compound according to any one of the preceding claims, wherein R₄, R₅, R₆, R₇, R₉, R₁₀, R₁₁, R₁₂, R₄', R₅', R₆', R₇', R₉', R₁₀', R₁₁', and R₁₂' are each independently selected from H, halogen, nitro, and cyano.

7. The compound according to any of the preceding claims selected from the compounds of formula (II) and (III) 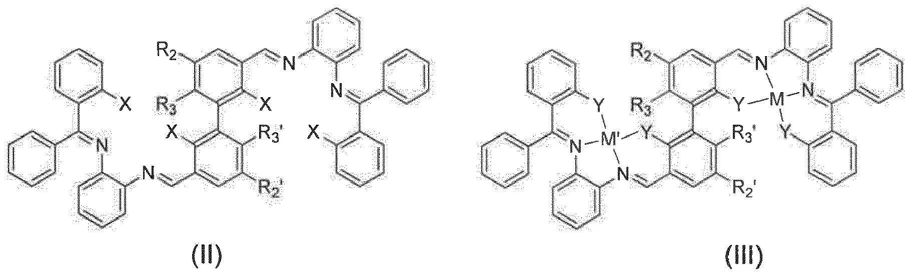 wherein:
M and M' are each independently selected from Ni(II), Cu(II), Sc(III), Fe(II), Fe(III), Ca(II), Co(II), Co(III), Os(III), Os(IV), AI(III), Mn(IV), Mn(III), Mn(II), Cr(III), Ti(II), Ti(IV), Zr(IV), Hf(IV), W(IV), W(V), Ta(IV), Ta(V), Pd(II), Pt(II), Pt(IV), Ir(III), Rh(III), Ru(II), Ru(III), Re(IV), In(II), In(III), Ga(III), Sn(II), Sn(IV), V(IV), V(V), Nb(V), Au(III), Mg(II), Cd(II), Ce(III), Ce(IV), Eu(III), Yb(III), Gd(III), and Zn(II);
each X is independently selected from OH and SH;
each Y is independently selected from O and S; and
R₂, R₃, R₂' and R₃' are independently selected from hydrogen, halogen, cyano and nitro; provided that one or more of the pairs of groups R₂ and R₃, R₂' and R₃', together with the carbon atoms to which they are attached optionally form a phenyl ring or a 5 or 6 membered heteroaromatic ring having one or more heteroatoms selected from O, N and S; wherein one or more of the hydrogen atoms of the phenyl or heteroaromatic ring is optionally replaced by a radical selected from halogen, nitro and cyano.

8. The compound according to claim 7, wherein R₂, R₃, R₂' and R₃' are each hydrogen; or the two pairs of R₂ and R₃ and R₂' and R₃' together with the carbon atoms to which they are attached each form a phenyl ring.

9. The compound according to claim 8, wherein
M and M' are the same and are selected from Ni(II), Cu(II) and Zn(II); and X is OH and Y is O.

10. A composition comprising:
a compound according to any one of the preceding claims or mixtures thereof; and
a carbonaceous material selected from amorphous carbon, graphene, graphite, fullerenes, carbon nanowires, carbon nanotubes and mixtures thereof, the carbonaceous material optionally having one or more functional groups selected from carboxy, halogen, hydroxy, alkoxy, siloxy, esters and amine.

11. The composition according to claim 10, further comprising a polymer or gel component.

12. The composition according to claim 11, comprising a polymer component which has a solubility in halogenated solvent of at least one gram of polymer per litre of solvent.

13. The composition according to claim 11 or 12, wherein the polymer is selected from linear low density polyethylene, polyethylene acrylates, poly(methyl methacrylate), poly(vinyl alcohol), polystyrene brominated, polyethylacrylate, polyvinyl acetate, polyvinyl nitrate, polyvinyl chloride, aromatic-aliphatic polyurethanes, aromatic polyurethanes, aliphatic polyurethanes, poly(3,4-ethylenedioxythiophene), poly(3,4-ethylenedioxythiophene)-tetramethacrylate, leucoemeraldine, emeraldine salt, emeraldine base, (per)nigraniline, trans-polyacetylene, cis-polyacetylene, aromatic-aliphatic polyketones, aromatic polyketones, aliphatic polyketones, polyether ether ketone, aromatic-aliphatic polypyrroles, aromatic polypyrroles and aliphatic polypyrroles, polyamide 46, polyamide 6, polyamide 66, polyamide 610, polyamide 612, polyamide 10, polyamide 11, polyamide 12, polyamide 6-6T, polyamide 6-61, polyamide 6T-6I, poly-oxydiphenylene-pyromellitimide, polydimethylsiloxane, aromatic-aliphatic polysiloxanes, aromatic polysiloxanes, poly(butylene succinate), poly(trimethylene terephthalate), polybutylene terephthalate, polyethylene terephthalate glycol, polyethylene terephthalate, polyhydroxybutyrate, polyhydroxyvalerate, polylactic acid, polyoxymethylene, polyphenylene oxide, polyphenylene sulphide, polyaspartic acid, ethylene acrylic acid sodium ionomer, poly(styrenesulfonate), PEDOT:PSS, acrylonitrile-butadiene-styrene copolymer, acrylic copolymers, acrylonitrile-styrene-acrylate copolymer, cyclic olefin copolymers, cyclic olefin polymers, ethylene acrylic ester copolymer, ethylene acrylic acid copolymer, ethylene epoxyacrylate copolymer, ethylene ethylacrylate copolymer, ethylene vinyl acetate copolymer, poly(butylene adipate-co-terephthalate), poly(ether-block-amides), poly(ethylene-co-methacrylic acid), methyl methacrylate copolymers, methacrylate copolymers, styrene acrylonitrile copolymer, polyvinyl chloride acetate, polymethylmethacrylate, polystyrene, styrene-butadiene block copolymer, bisphenol-A polycarbonate, high density polyethylene, low density polyethylene, poly ethylene acrylates, polyacetic acid, polyacrylic acid, polyacrylate, polyacrylonitrile, polyamide, polybutadiene, polybutylene, polybutylene adipate, polycarbonate, aromatic-aliphatic polycarbonates, aromatic polycarbonates, aliphatic polycarbonates, polyphenylsulphone, polyethersulfone, polyethylene, polymethylpentene, polyoxymethylene acetal polymer, polyphenylene sulfide, polypropylene, polysulphone, polytetrafluoroethylene, polyurethane, polyhydroxybutyrate, PEDOT, polypyrrole, polyaniline, polyacetylenes, mixtures, blends and co-polymers thereof.

14. A composition according to any one of claims 11 to 13, wherein the amount of carbonaceous material in the composition is from 0.00001 to 30 wt.% and the amount of polymer or gel component in the composition is at least 70 wt.%.

15. A method for preparing a composition according to any one of claims 10 to 14, the method comprising the steps of: preparing a mixture of carbonaceous material with a compound according to any one of claims 1 to 9 and optionally also a polymer or gel component, in a solvent; and eliminating the solvent to provide the composition.
